(19) Europäisches Patentamt
European Patent Office
Office européen des brevets

(11) EP 4 212 162 A1

(12) **EUROPEAN PATENT APPLICATION**
published in accordance with Art. 153(4) EPC

(43) Date of publication:
**19.07.2023 Bulletin 2023/29**

(21) Application number: **21866911.7**

(22) Date of filing: **14.09.2021**

(51) International Patent Classification (IPC):
***A61K 31/7088*** *(2006.01)* ***A61K 47/10*** *(2017.01)*
***A61K 47/18*** *(2017.01)* ***A61K 47/24*** *(2006.01)*
***A61K 47/28*** *(2006.01)* ***A61K 48/00*** *(2006.01)*
***A61P 25/00*** *(2006.01)* ***A61P 43/00*** *(2006.01)*
***C12N 15/88*** *(2006.01)*

(52) Cooperative Patent Classification (CPC):
(C-Sets available)
**A61K 9/0019; A61K 9/1272; A61K 31/7088; A61K 47/14; A61K 47/24; A61P 25/00; A61P 43/00; C12N 15/111; C12N 15/88;**
C12N 15/1137; C12N 2310/11; C12N 2310/315; C12N 2310/321; C12N 2310/3341; C12N 2310/341

(Cont.)

(86) International application number:
**PCT/JP2021/033635**

(87) International publication number:
**WO 2022/054955 (17.03.2022 Gazette 2022/11)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **14.09.2020 JP 2020153509**

(71) Applicant: **FUJIFILM Corporation**
**Tokyo 106-8620 (JP)**

(72) Inventors:
- **YAMADA Naoki**
  **Ashigarakami-gun, Kanagawa 258-8577 (JP)**
- **KANEUMI Shun**
  **Ashigarakami-gun, Kanagawa 258-8577 (JP)**
- **SUZUKI Keiko**
  **Ashigarakami-gun, Kanagawa 258-8577 (JP)**
- **KASAGI Noriyuki**
  **Ashigarakami-gun, Kanagawa 258-8577 (JP)**
- **NAKAMURA Naoto**
  **Ashigarakami-gun, Kanagawa 258-8577 (JP)**

(74) Representative: **Klunker IP**
**Patentanwälte PartG mbB**
**Destouchesstraße 68**
**80796 München (DE)**

(54) **LIPID COMPOSITION**

(57) An object of the present invention is to provide a lipid composition capable of achieving excellent nucleic acid delivery. According to the present invention, there is provided a lipid composition containing a lipid represented by Formula (1) or a salt thereof, a nucleic acid, at least one non-cationic lipid, and a lipid represented by $R^{51}$-L-$(OCH_2CH_2)_n$-O-$R^{52}$ (in the formula, $R^{51}$ represents a hydrocarbon group having 6 to 30 carbon atoms, L represents -CO- or a single bond, $R^{52}$ represents a hydrogen atom or an alkyl group having 1 to 6 carbon atoms, and n represents an integer of 10 to 150).



Processed by Luminess, 75001 PARIS (FR)

(Cont. next page)

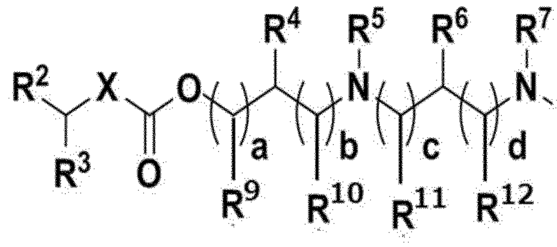

(1)

In the formula, each symbol means the definition described in the description.

FIG. 1

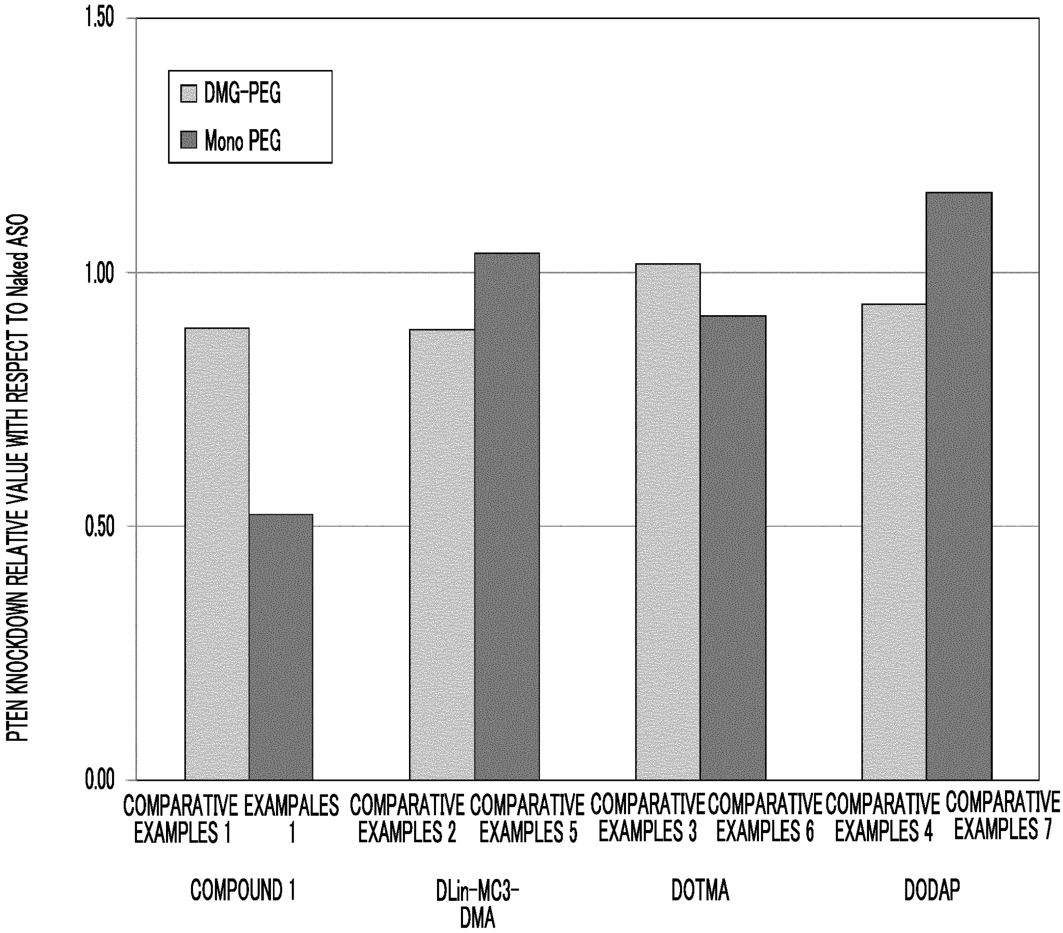


(52) Cooperative Patent Classification (CPC): (Cont.)

C-Sets
C12N 2310/321, C12N 2310/3525

**Description**

Field of the Invention

The present invention relates to a lipid composition containing a nucleic acid and a lipid.

Description of the Related Art

Nucleic acid drugs have a clear mechanism of action on diseases, have few side effects, and are expected as next-generation drugs, and nucleic acid drugs are being actively developed. As one technique for delivering a nucleic acid to a cell, a method of encapsulating the nucleic acid in a liposome or a lipid particle and administering the nucleic acid is known. In this technique, as the lipids, lipids having a substituent such as an amino group, which turn into cations at a low pH, are used and appropriate charge is applied to the particles for the delivery of nucleic acids.

As a compound to be incorporated into the lipid particles, Patent Document 1 and Patent Document 2disclose a compound having an ester group, an acetal group, or the like as a linking group that links an aliphatic group to an amino group. Patent Document 3 discloses a compound having a vinyloxy group, an amide group, an oxime group, or the like as a linking group that links an aliphatic group to an amino group. Patent Document 4 describes lipid nanoparticles containing DLin-MC3-DMA that is a cationic lipid. Patent Document 5 describes cationic lipids that are useful for delivering a drug to cells and tissues.

In Patent Document 6, there are studies on a type and a composition ratio of a lipid compound to be combined in a case of producing nucleic acid-containing particles.

In addition, Patent Document 7 describes lipid particles containing a lipid compound having a predetermined structure. Furthermore, Patent Document 8 describes a lipid having a polyethylene glycol structure.

Prior Art Documents

Patent Documents

Patent Document 1: WO2010/054401A
Patent Document 2: WO2010/144740A
Patent Document 3: WO2010/054405A
Patent Document 4: US2013-0245107A
Patent Document 5: WO2015/095340A
Patent Document 6: WO2009/127060A
Patent Document 7: WO2019/235635A
Patent Document 8: JP2019-504002A

**SUMMARY OF THE INVENTION**

There is a search for lipid compositions capable of functioning as a vector for delivering a nucleic acid, and development of a lipid composition capable of achieving excellent nucleic acid delivery is still desired. An object of the present invention is to provide a lipid composition capable of achieving excellent nucleic acid delivery.

In order to achieve the above object, the inventors of the present invention conducted intensive studies. As a result, the inventors have accomplished the present invention by finding that a lipid composition prepared by using a lipid represented by Formula (1) and a lipid in which a hydrocarbon chain and a polyethylene glycol structure are bonded, exhibits excellent nucleic acid delivery. According to the present invention, the following inventions are provided.

<1> A lipid composition containing a lipid represented by Formula (1) or a salt thereof, a nucleic acid, at least one non-cationic lipid, and a lipid represented by $R^{51}$-L-$(OCH_2CH_2)_n$-O-$R^{52}$ (in the formula, $R^{51}$ represents a hydrocarbon group having 6 to 30 carbon atoms, L represents -CO- or a single bond, $R^{52}$ represents a hydrogen atom or an alkyl group having 1 to 6 carbon atoms, and n represents an integer of 10 to 150).

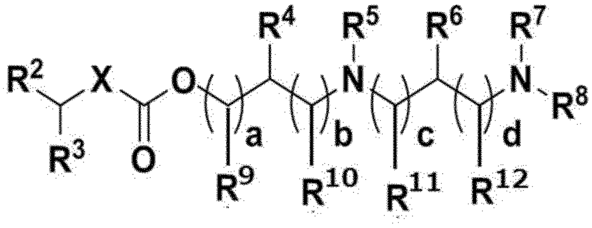

(1)

In the formula, X represents $-NR^1-$ or -O-,

$R^1$ represents a hydrogen atom, a hydrocarbon group having 6 to 24 carbon atoms, or a group represented by $R^{21}-L^1-R^{22}-$, where $R^{21}$ represents a hydrocarbon group having 1 to 24 carbon atoms, $L^1$ represents -O(CO)O-, -O(CO)-, -(CO)O-, -O-, or a group represented by the following structural formula, and

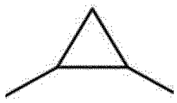

$R^{22}$ represents a divalent hydrocarbon linking group having 1 to 18 carbon atoms,
$R^2$ and $R^3$ each independently represent a hydrogen atom, a hydrocarbon group having 3 to 24 carbon atoms, or a group represented by $R^{31}-L^2-R^{32}-$, where $R^{31}$ represents a hydrocarbon group having 1 to 24 carbon atoms, $L^2$ represents -O(CO)O-, -O(CO)-, -(CO)O-, -O-, or a group represented by the following structural formula, and

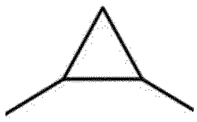

$R^{32}$ represents a divalent hydrocarbon linking group having 1 to 18 carbon atoms,
$R^4$, $R^5$, $R^6$, $R^7$, $R^8$, $R^9$, $R^{10}$, $R^{11}$, and $R^{12}$ each independently represent a hydrogen atom or an alkyl group having 1 to 18 carbon atoms which may be substituted,
groups in any one or more pairs among $R^4$ and $R^5$, $R^{10}$ and $R^5$, $R^5$ and $R^{12}$, $R^4$ and $R^6$, $R^5$ and $R^6$, $R^6$ and $R^7$, $R^6$ and $R^{10}$, $R^{12}$ and $R^7$, and $R^7$ and $R^8$ may be linked to each other to form a 4- to 7-membered ring which may contain an O atom,
a substituent on the alkyl group having 1 to 18 carbon atoms which may be substituted is a hydroxyl group, a carboxyl group, an amino group represented by $-NR^{45}R^{46}$, a substituted or unsubstituted aryl group, a substituted or unsubstituted heteroaryl group, or a group represented by $-O(CO)O-R^{41}$, $-O(CO)-R^{42}$, $-(CO)O-R^{43}$, or $-O-R^{44}$, where $R^{41}$, $R^{42}$, $R^{43}$, $R^{44}$, $R^{45}$ and $R^{46}$ each independently represent a hydrocarbon group having 1 to 18 carbon atoms,
a substituent on the substituted or unsubstituted aryl group and on the substituted or unsubstituted heteroaryl group is an alkyl group having 1 to 18 carbon atoms, a hydroxyl group, a carboxyl group, an amino group represented by $-NR^{45}R^{46}$, or a group represented by $-O(CO)O-R^{41}$, $-O(CO)-R^{42}$, $-(CO)O-R^{43}$, or $-O-R^{44}$, where $R^{41}$, $R^{42}$, $R^{43}$, $R^{44}$, $R^{45}$, and $R^{46}$ each independently represent a hydrocarbon group having 1 to 18 carbon atoms, and
a, b, c, and d each independently represent an integer of 0 to 3, provided that a + b is 1 or more, and c + d is 1 or more.

<2> The lipid composition described in <1>, in which the lipid represented by Formula (1) is a lipid represented by Formula (2).

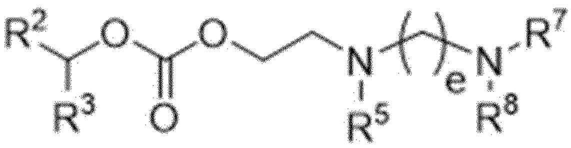

(2)

In the formula,

$R^2$ and $R^3$ are each independently a hydrocarbon group having 3 to 24 carbon atoms which contains one or more unsaturated bonds,
$R^2$ and $R^3$ are each independently a group represented by $R^{31}$-$L^2$-$R^{32}$-, or
one of $R^2$ and $R^3$ is a group represented by $R^{31}$-$L^2$-$R^{32}$-, and the other is a hydrocarbon group having 3 to 24 carbon atoms,
where $R^{31}$ represents a hydrocarbon group having 1 to 24 carbon atoms,
$L^2$ represents -O(CO)O-, -O(CO)-, -(CO)O-, -O-, or a group represented by the following structural formula, and

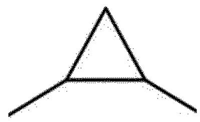

$R^{32}$ represents a divalent hydrocarbon linking group having 1 to 18 carbon atoms,
$R^5$ represents an alkyl group having 1 to 18 carbon atoms which may be substituted with -O(CO)-$R^{42}$ or -(CO)O-$R^{43}$, where $R^{42}$ and $R^{43}$ each independently represent a hydrocarbon group having 1 to 18 carbon atoms,
$R^7$ and $R^8$ each independently represent an alkyl group having 1 to 4 carbon atoms, and
e represents 2 or 3.

<3> The lipid composition described in <1> or <2>, in which the content of the lipid represented by Formula (1) is 30 to 70 mol% with respect to the total amount of the lipid.
<4> The lipid composition described in any one of <1> to <3>, in which the non-cationic lipid is a lipid selected from a non-ionic lipid and a zwitterionic lipid.
<5> The lipid composition described in <4>, in which the non-cationic lipid includes at least one non-ionic lipid and at least one zwitterionic lipid.
<6> The lipid composition described in <4> or <5>, in which the non-ionic lipid is sterols.
<7> The lipid composition described in <6>, in which the sterols are phytosterol or cholesterol.
<8> The lipid composition described in any one of <5> to <7>, in which the content of the non-ionic lipid is 10 to 60 mol% with respect to the total amount of the lipid.
<9> The lipid composition described in <4> or <5>, in which the zwitterionic lipid is a phospholipid.
<10> The lipid composition described in any one of <4> to <9>, in which the content of the zwitterionic lipid is 1 to 30 mol% with respect to the total amount of the lipid.
<11> The lipid composition described in any one of <1> to <10>, in which $R^{51}$ represents a hydrocarbon group having 8 to 26 carbon atoms.
<12> The lipid composition described in any one of <1> to <11>, in which the content of the lipid represented by $R^{51}$-L-$(OCH_2CH_2)_n$-O-$R^{52}$ is 0. 1 to 10 mol% with respect to the total amount of the lipid.
<13> The lipid composition described in any one of <1> to <12>, in which the weight ratio of the lipid to the nucleic acid is 5 to 100.
<14> The lipid composition described in any one of <1> to <13>, further including a pharmaceutically acceptable carrier.
<15> The lipid composition described in <14>, in which the lipid composition is a pharmaceutical composition for introducing a nucleic acid into a cell.
<16> The lipid composition described in <15>, in which the lipid composition is a pharmaceutical composition for nucleic acid delivery in vivo.
<17> The lipid composition described in <16>, in which the lipid composition is a pharmaceutical composition for nucleic acid delivery in vivo by topical administration.
<18> The lipid composition described in <17>, in which the topical administration in vivo is administration to the central nervous system.
<19> The lipid composition described in any one of <1> to <13>, in which the lipid composition is a nucleic acid delivery carrier.

The lipid composition according to the embodiment of the present invention can achieve excellent nucleic acid delivery.

## BRIEF DESCRIPTION OF THE DRAWINGS

Fig. 1 shows the comparison result of evaluations of PTEN mRNA knockdown in vitro in a case where the type of a cationic lipid is changed.
Fig. 2 shows the comparison result of evaluations of PTEN mRNA knockdown in vitro in a case where the type of a phospholipid is changed.
Fig. 3 shows the comparison result of evaluations of PTEN mRNA knockdown in vitro in a case where the mol% of a PEG lipid is changed.
Fig. 4 shows the comparison result of evaluations of PTEN mRNA knockdown in vitro in a case where the lipid/RNA ratio is changed.
Fig. 5 shows the results of evaluation of PTEN mRNA knockdown in an intracerebroventricular administration.
Fig. 6 shows the results of evaluation of PTEN mRNA knockdown in an intracerebroventricular administration.

## DESCRIPTION OF THE PREFERRED EMBODIMENTS

Hereinafter, the present invention will be specifically described.

In the present specification, "to" shows a range including numerical values described before and after "to" as a minimum value and a maximum value respectively.

The lipid composition of the embodiment of the present invention contains a lipid represented by Formula (1) or a salt thereof, a nucleic acid, at least one non-cationic lipid, and a lipid represented by $R^{51}$-L-$(OCH_2CH_2)_n$-O-$R^{52}$ (in the formula, $R^{51}$ represents a hydrocarbon group having 6 to 30 carbon atoms, L represents -CO- or a single bond, $R^{52}$ represents a hydrogen atom or an alkyl group having 1 to 6 carbon atoms, and n represents an integer of 10 to 150). The fact that by using the lipid represented by Formula (1) or a salt thereof in combination with the lipid represented by $R^{51}$-L-$(OCH_2CH_2)_n$-O-$R^{52}$, the effect of introduction of a nucleic acid into a cell can be effectively exhibited, that is, the excellent nucleic acid delivery can be achieved, is a finding found for the first time in the present invention. In a case where DLin-MC3-DMA is used instead of the lipid represented by Formula (1) or a salt thereof, or a polyethylene glycol lipid other than the lipid represented by $R^{51}$-L-$(OCH_2CH_2)_n$-O-$R^{52}$ is used, the effect of the present invention is not achieved. In the present invention, an unexpected effect can be achieved by using the above-described two types of specific lipids in combination.

<Lipid represented by Formula (1) or salt thereof>

In the present invention, a lipid represented by Formula (1) or a salt thereof is used.

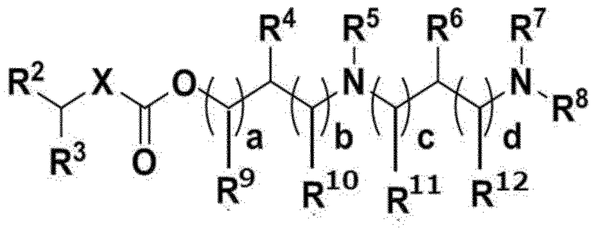

(1)

in the formula,

X represents -$NR^1$- or -O-,
$R^1$ represents a hydrogen atom, a hydrocarbon group having 6 to 24 carbon atoms, or a group represented by $R^{21}$-$L^1$-$R^{22}$-, where $R^{21}$ represents a hydrocarbon group having 1 to 24 carbon atoms, $L^1$ represents -O(CO)O-, -O(CO)-, -(CO)O-, -O-, or a group represented by the following structural formula, and

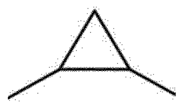

$R^{22}$ represents a divalent hydrocarbon linking group having 1 to 18 carbon atoms,

$R^2$ and $R^3$ each independently represent a hydrogen atom, a hydrocarbon group having 3 to 24 carbon atoms, or a group represented by $R^{31}$-$L^2$-$R^{32}$-, where $R^{31}$ represents a hydrocarbon group having 1 to 24 carbon atoms, $L^2$ represents -O(CO)O-, -O(CO)-, -(CO)O-, -O-, or a group represented by the following structural formula, and

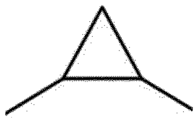

$R^{32}$ represents a divalent hydrocarbon linking group having 1 to 18 carbon atoms,
$R^4$, $R^5$, $R^6$, $R^7$, $R^8$, $R^9$, $R^{10}$, $R^{11}$, and $R^{12}$ each independently represent a hydrogen atom or an alkyl group having 1 to 18 carbon atoms which may be substituted,
groups in any one or more pairs among $R^4$ and $R^5$, $R^{10}$ and $R^5$, $R^5$ and $R^{12}$, $R^4$ and $R^6$, $R^5$ and $R^6$, $R^6$ and $R^7$, $R^6$ and $R^{10}$, $R^{12}$ and $R^7$, and $R^7$ and $R^8$ may be linked to each other to form a 4- to 7-membered ring which may contain an O atom,
a substituent on the alkyl group having 1 to 18 carbon atoms which may be substituted is a hydroxyl group, a carboxyl group, an amino group represented by -$NR^{45}R^{46}$, a substituted or unsubstituted aryl group, a substituted or unsubstituted heteroaryl group, or a group represented by -O(CO)O-$R^{41}$, -O(CO)-$R^{42}$, -(CO)O-$R^{43}$, or -O-$R^{44}$, where $R^{41}$, $R^{42}$, $R^{43}$, $R^{44}$, $R^{45}$ and $R^{46}$ each independently represent a hydrocarbon group having 1 to 18 carbon atoms,
a substituent on the substituted or unsubstituted aryl group and on the substituted or unsubstituted heteroaryl group is an alkyl group having 1 to 18 carbon atoms, a hydroxyl group, a carboxyl group, an amino group represented by -$NR^{45}R^{46}$, or a group represented by -O(CO)O-$R^{41}$, -O(CO)-$R^{42}$, -(CO)O-$R^{43}$, or -O-$R^{44}$, where $R^{41}$, $R^{42}$, $R^{43}$, $R^{44}$, $R^{45}$, and $R^{46}$ each independently represent a hydrocarbon group having 1 to 18 carbon atoms, and
a, b, c, and d each independently represent an integer of 0 to 3, provided that a + b is 1 or more, and c + d is 1 or more.

As the hydrocarbon group having 6 to 24 carbon atoms that is represented by $R^1$ and the hydrocarbon group having 3 to 24 carbon atoms that is represented by $R^2$ and $R^3$, an alkyl group, an alkenyl group, or an alkynyl group is preferable, and an alkyl group or an alkenyl group is more preferable. The alkyl group having 6 to 24 carbon atoms and the alkyl group having 3 to 24 carbon atoms may be linear or branched or may be chain-like or cyclic. The alkyl group having 6 to 24 carbon atoms is preferably an alkyl group having 6 to 20 carbon atoms, and the alkyl group having 3 to 24 carbon atoms is more preferably an alkyl group having 6 to 20 carbon atoms. Specifically, examples thereof include a hexyl group, a heptyl group, an octyl group, a nonyl group, a decyl group, an undecyl group, a dodecyl group, a tridecyl group, a trimethyldodecyl group (preferably a 3,7,11-trimethyldodecyl group), a tetradecyl group, a pentadecyl group, a hexadecyl group, a tetramethylhexadecyl group (preferably a 3,7,11,15-tetramethylhexadecyl group), a heptadecyl group, an octadecyl group, a nonadecyl group, and an icosyl group. The alkenyl group having 6 to 24 carbon atoms and the alkenyl group having 3 to 24 carbon atoms may be linear or branched or may be chain-like or cyclic. The alkenyl group having 6 to 24 carbon atoms is preferably an alkenyl group having 6 to 20 carbon atoms, and the alkenyl group having 3 to 24 carbon atoms is more preferably an alkenyl group having 6 to 20 carbon atoms. Specifically, examples thereof include a hexenyl group, a heptenyl group, an octenyl group, a nonenyl group, a decenyl group, an undecenyl group, a dodecenyl group, a dodecadienyl group, a tridecenyl group, a tetradecenyl group, a pentadecenyl group, a hexadecenyl group (preferably a (Z)-hexadec-9-enyl group), a hexadecadienyl group, a heptadecenyl group (preferably a (Z)-heptadec-8-enyl group), a heptadecadienyl group (preferably an (8Z,11Z)-heptadeca-8,11-dienyl group), an octadecenyl group (preferably a (Z)-octadec-9-enyl group), an octadecadienyl group (preferably a (9Z,12Z)-octadeca-9,12-dienyl group), a nonadecenyl group, an icosenyl group (preferably a (Z)-icos-11-enyl group), and an icosadienyl group (preferably an (11Z,14Z)-icosa-11,14-dienyl group). The alkynyl group having 6 to 24 carbon atoms is preferably an alkynyl group having 6 to 20 carbon atoms, and the alkynyl group having 3 to 24 carbon atoms is more preferably an alkynyl group having 6 to 20 carbon atoms. Specifically, examples thereof include a hexynyl group, a heptynyl group, an octynyl group, a nonynyl group, a decynyl group, an undecynyl group, a dodecynyl group, a tetradecynyl group, a pentadecynyl group, a hexadecynyl group, a heptadecynyl group, and an octadecynyl group. All of the above alkenyl groups preferably have one double bond or two double bonds. All of the above alkynyl groups preferably have one triple bond or two triple bonds.

As hydrocarbon group having 1 to 24 carbon atoms that is represented by $R^{21}$ and $R^{31}$, an alkyl group having 10 to 24 carbon atoms, an alkenyl group having 10 to 24 carbon atoms, or an alkynyl group having 10 to 24 carbon atoms is preferable. The alkyl group having 10 to 24 carbon atoms may be linear or branched or may be chain-like or cyclic. The alkyl group having 10 to 24 carbon atoms is preferably an alkyl group having 12 to 24 carbon atoms. Specifically, examples thereof include a decyl group, an undecyl group, a dodecyl group, a tridecyl group, a trimethyldodecyl group (preferably a 3,7,11-trimethyldodecyl group), a tetradecyl group, a pentadecyl group, a hexadecyl group, a tetramethylhexadecyl group (preferably a 3,7,11,15-tetramethylhexadecyl group), a heptadecyl group, an octadecyl group, a 2-butylhexyl group, a 2-butyloctyl group, a 1-pentylhexyl group, a 2-pentylheptyl group, a 3-pentyloctyl group, a 1-hexylheptyl group, a 1-hexylnonyl group, a 2-hexyloctyl group, a 2-hexyldecyl group, a 3-hexylnonyl group, a 1-heptyloctyl group, a 2-heptylnonyl group, a 2-heptylundecyl group, a 3-heptyldecyl group, a 1-octylnonyl group, a 2-octyldecyl group, a 2-octyldodecyl group, a 3-octylundecyl group, a 2-nonylundecyl group, a 3-nonyldodecyl group, a 2-decyldodecyl group, a 2-decyltetradecyl group, a 3-decyltridecyl group, and a 2-(4,4-dimethylpentan-2-yl)-5,7,7-trimethyloctyl group. The alkenyl group having 10 to 24 carbon atoms may be linear or branched or may be chain-like or cyclic. Specifically, examples thereof include a decenyl group, an undecenyl group, a dodecenyl group, a dodecadienyl group, tridecenyl group (preferably a (Z)-tridec-8-enyl group), a tetradecenyl group (preferably a tetradec-9-enyl group), a pentadecenyl group (preferably a (Z)-pentadec-8-enyl group), a hexadecenyl group (preferably a (Z)-hexadec-9-enyl group), a hexadecadienyl group, a heptadecenyl group (preferably a (Z)-heptadec-8-enyl group), a heptadecadienyl group (preferably a (8Z,11Z)-heptadeca-8,11-dienyl group), an octadecenyl group (preferably a (Z)-octadec-9-enyl group), and an octadecadienyl group (preferably a (9Z,12Z)-octadeca-9,12-dienyl group). The alkynyl group having 10 to 24 carbon atoms may be linear or branched or may be chain-like or cyclic. Specifically, examples thereof include a decynyl group, an undecynyl group, a dodecynyl group, a tetradecynyl group, a pentadecynyl group, a hexadecynyl group, a heptadecynyl group, and an octadecynyl group. All of the above alkenyl groups preferably have one double bond or two double bonds. All of the above alkynyl groups preferably have one triple bond or two triple bonds.

As the divalent hydrocarbon linking group having 1 to 18 carbon atoms that is represented by $R^{22}$ and $R^{32}$, an alkylene group having 1 to 18 carbon atoms or an alkenylene group having 2 to 18 carbon atoms is preferable. The alkylene group having 1 to 18 carbon atoms may be linear or branched or may be chain-like or cyclic. The number of carbon atoms in the alkylene group having 1 to 18 carbon atoms is preferably 1 to 12, more preferably 1 to 10, and still more preferably 2 to 10. Specifically, examples thereof include a methylene group, an ethylene group, a trimethylene group, a tetramethylene group, a pentamethylene group, a hexamethylene group, a heptamethylene group, an octamethylene group, a nonamethylene group, a decamethylene group, an undecamethylene group, and a dodecamethylene group. The alkenylene group having 2 to 18 carbon atoms may be linear or branched or may be chain-like or cyclic. The number of carbon atoms in the alkenylene group having 2 to 18 carbon atoms is preferably 1 to 12, and more preferably 2 to 10.

As a preferred range of $L^1$, -O(CO)O-, -O(CO)-, or -(CO)O- is preferable and -O(CO)- or -(CO)O- is more preferable.

As a preferred range of $L^2$, -O(CO)O-, -O(CO)-, or -(CO)O- is preferable and -O(CO)- or -(CO)O- is more preferable.

The alkyl group having 1 to 18 carbon atoms which may be substituted and which represented by $R^4$, $R^6$, $R^9$, $R^{10}$, $R^{11}$, and $R^{12}$ may be linear or branched or may be chain-like or cyclic. The number of carbon atoms in the alkyl group having 1 to 18 carbon atoms is preferably 1 to 12. Specifically, examples thereof include a methyl group, an ethyl group, a propyl group, an isopropyl group, a cyclopropyl group, a butyl group, an isobutyl group, a tert-butyl group, a cyclobutyl group, a pentyl group, a cyclopentyl group, a hexyl group, a cyclohexyl group, a heptyl group, an octyl group, a nonyl group, a decyl group, an undecyl group, and a dodecyl group. In a case where the alkyl group has a substituent, as the substituent, a hydroxyl group, a carboxyl group, or a group represented by -O(CO)O-$R^{41}$, -O(CO)-$R^{42}$, -(CO)O-$R^{43}$, or -O-$R^{44}$ is preferable, and a group represented by -O(CO)-$R^{42}$ or -(CO)O-$R^{43}$ is more preferable.

The alkyl group having 1 to 18 carbon atoms which may be substituted and which represented by $R^5$, R', and $R^8$ may be linear or branched or may be chain-like or cyclic. The number of carbon atoms in the alkyl group having 1 to 18 carbon atoms is preferably 1 to 12 and more preferably 1 to 8. Specifically, examples thereof include a methyl group, an ethyl group, a propyl group, an isopropyl group, a cyclopropyl group, a butyl group, an isobutyl group, a tert-butyl group, a cyclobutyl group, a pentyl group, a cyclopentyl group, a hexyl group, a cyclohexyl group, a heptyl group, an octyl group, a nonyl group, a decyl group, an undecyl group, and a dodecyl group. In a case where the alkyl group has a substituent, as the substituent, a hydroxyl group, a carboxyl group, or a group represented by -O(CO)O-$R^{41}$, -O(CO)-$R^{42}$, -(CO)O-$R^{43}$, or -O-$R^{44}$ is preferable, and a group represented by -O(CO)-$R^{42}$ or -(CO)O-$R^{43}$ is more preferable.

Examples of the 4- to 7-membered ring which may contain an O atom include an azetidine ring, a pyrrolidine ring, a piperidine ring, a morpholine ring, and an azepane ring. The 4- to 7-membered ring is preferably a 6-membered ring and is preferably a piperidine ring or a morpholine ring.

In a case where the alkyl group having 1 to 18 carbon atoms which is represented by $R^4$, $R^5$, $R^6$, $R^7$, $R^8$, $R^9$, $R^{10}$, $R^{11}$, and $R^{12}$ and which may be substituted has a substituted or unsubstituted aryl group as a substituent, the number of carbon atoms in the aryl group is preferably 6 to 22, more preferably 6 to 18, and still more preferably 6 to 10. Specifically, examples of the aryl group include a phenyl group, a naphthyl group, an anthracenyl group, and a phenanthrenyl group. As the substituent on the aryl group, an alkyl group having 1 to 18 carbon atoms, a hydroxyl group, a carboxyl group, an amino group represented by -$NR^{45}R^{46}$, or a group represented by -O(CO)O-$R^{41}$, -O(CO)-$R^{42}$, -(CO)O-$R^{43}$, or -O-$R^{44}$ is preferable, and a hydroxyl group or a carboxyl group is more preferable. Specifically, examples of the substituted aryl group include a hydroxyphenyl group, and a carboxyphenyl group.

In a case where the alkyl group having 1 to 18 carbon atoms which is represented by $R^4$, $R^5$, $R^6$, $R^7$, $R^8$, $R^9$,

$R^{10}$, $R^{11}$, and $R^{12}$ and which may be substituted has a substituted or unsubstituted heteroaryl group as a substituent, the number of carbon atoms in the heteroaryl group is preferably 1 to 12, and more preferably 1 to 6. Specifically, examples of the heteroaryl group include a pyridyl group, a pyrazolyl group, an imidazolyl group, a benzimidazolyl group, a thiazolyl group, and an oxazolyl group. As the substituent on the heteroaryl group, an alkyl group having 1 to 18 carbon atoms, a hydroxyl group, a carboxyl group, an amino group represented by $-NR^{45}R^{46}$, or a group represented by $-O(CO)O-R^{41}$, $-O(CO)-R^{42}$, $-(CO)O-R^{43}$, or $-O-R^{44}$ is preferable, and a hydroxyl group or a carboxyl group is more preferable. Specifically, examples of the substituted or unsubstituted heteroaryl group include a hydroxypyridyl group, a carboxypyridyl group, and a pyridonyl group.

As hydrocarbon group having 1 to 18 carbon atoms that is represented by $R^{41}$, $R^{42}$, $R^{43}$, $R^{44}$, $R^{45}$, and $R^{46}$, an alkyl group having 1 to 18 carbon atoms, an alkenyl group having 2 to 18 carbon atoms, or an alkynyl group having 2 to 18 carbon atoms is preferable, and an alkyl group having 1 to 18 carbon atoms or an alkenyl group having 2 to 18 carbon atoms is more preferable. The alkyl group having 1 to 18 carbon atoms may be linear or branched or may be chain-like or cyclic. The number of carbon atoms in the alkyl group having 1 to 18 carbon atoms is preferably 3 to 18, and more preferably 5 to 18. Specifically, examples thereof include a propyl group, an isopropyl group, a cyclopropyl group, a butyl group, an isobutyl group, a tert-butyl group, a cyclobutyl group, a pentyl group, a cyclopentyl group, a hexyl group, a cyclohexyl group, a heptyl group, an octyl group, a nonyl group, a decyl group, an undecyl group, a dodecyl group, a tridecyl group, a trimethyldodecyl group (preferably a 3,7,11-trimethyldodecyl group), a tetradecyl group, a pentadecyl group, a hexadecyl group, a heptadecyl group, and an octadecyl group. The alkenyl group having 2 to 18 carbon atoms may be linear or branched or may be chain-like or cyclic. The number of carbon atoms in the alkenyl group having 2 to 18 carbon atoms is preferably 3 to 18, and more preferably 5 to 18. Specifically, examples thereof include an allyl group, a prenyl group, a pentenyl group, a hexenyl group, a heptenyl group, an octenyl group, a nonenyl group (preferably a (Z)-2-nonenyl group or an (E)-2-nonenyl group), a decenyl group, an undecenyl group, a dodecenyl group, a dodecadienyl group, a tridecenyl group (preferably a (Z)-tridec-8-enyl group), a tetradecenyl group (preferably a tetradec-9-enyl group), a pentadecenyl group (preferably a (Z)-pentadec-8-enyl group), a hexadecenyl group (preferably a (Z)-hexadec-9-enyl group), a hexadecadienyl group, a heptadecenyl group (preferably a (Z)-heptadec-8-enyl group), a heptadecadienyl group (preferably a (8Z,11Z)-heptadeca-8,11-dienyl group), an octadecenyl group (preferably a (Z)-octadec-9-enyl group), and an octadecadienyl group (preferably a (9Z,12Z)-octadeca-9,12-dienyl group). The alkynyl group having 2 to 18 carbon atoms may be linear or branched or may be chain-like or cyclic. The number of carbon atoms in the alkynyl group having 2 to 18 carbon atoms is preferably 3 to 18, and more preferably 5 to 18. Specifically, examples thereof include a propargyl group, a butynyl group, a pentynyl group, a hexynyl group, a heptynyl group, an octynyl group, a nonynyl group, a decynyl group, an undecynyl group, a dodecynyl group, a tetradecynyl group, a pentadecynyl group, a hexadecynyl group, a heptadecynyl group, and an octadecynyl group.

In a case where X represents $-NR^1-$, it is preferable that $R^1$ is a hydrocarbon group having 6 to 24 carbon atoms or a group represented by $R^{21}-L^1-R^{22}-$. In this case, it is preferable that one of $R^2$ and $R^3$ is a hydrogen atom and the other is a hydrocarbon group having 6 to 24 carbon atoms or a group represented by $R^{31}-L^2-R^{32}-$.

In a case where X represents -O-, it is preferable that $R^2$ and $R^3$ are each independently a hydrocarbon group having 6 to 24 carbon atoms or a group represented by $R^{31}-L^2-R^{32}-$.

It is preferable that $R^4$, $R^6$, $R^9$, $R^{10}$, $R^{11}$, and $R^{12}$ are a hydrogen atom.

It is preferable that $R^5$ is a hydrogen atom, an alkyl group having 1 to 18 carbon atoms, an alkyl group having 1 to 18 carbon atoms which may be substituted with $-O(CO)-R^{42}$ or $-(CO)O-R^{43}$, an alkyl group having 1 to 18 carbon atoms which may be substituted with an aryl group, or an alkyl group having 1 to 18 carbon atoms which may be substituted with a hydroxyl group. In a case where $R^5$ is an alkyl group, $R^5$ may be linked to $R^4$, $R^6$, $R^{10}$, and $R^{12}$ to form a ring which may contain an O atom. Particularly, it is preferable that $R^5$ is an alkyl group having 1 to 18 carbon atoms, an alkyl group having 1 to 18 carbon atoms which may be substituted with $-O(CO)-R^{42}$ or $-(CO)O-R^{43}$, an alkyl group having 1 to 12 carbon atoms which may be substituted with an aryl group, or an alkyl group having 1 to 8 carbon atoms which may be substituted with a hydroxyl group, and more preferable that $R^5$ is an alkyl group having 1 to 18 carbon atoms or an alkyl group having 1 to 18 carbon atoms which may be substituted with $-O(CO)-R^{42}$ or $-(CO)O-R^{43}$.

It is preferable that $R^7$ and $R^8$ are each independently a hydrogen atom, a hydrocarbon group having 1 to 18 carbon atoms, an alkyl group having 1 to 18 carbon atoms which may be substituted with $-O(CO)-R^{42}$ or $-(CO)O-R^{43}$, an alkyl group having 1 to 8 carbon atoms which may be substituted with an aryl group, or an alkyl group having 1 to 8 carbon atoms which may be substituted with a hydroxyl group, or alternatively, R' and $R^8$ are linked to each other to form a 4- to 7-membered ring which may contain an O atom.

$R^5$ is not linked to $R^7$ or $R^8$ and does not form a ring with $R^7$ or $R^8$.

a + b is preferably 1 or 2, and more preferably 1. c + d is preferably 1 or 2, and more preferably 1.

In a preferred embodiment, the lipid represented by Formula (1) is a lipid represented by Formula (2).

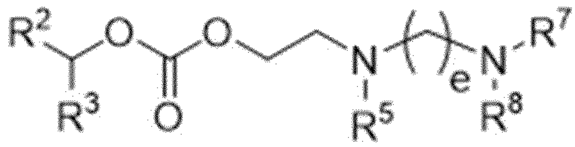

(2)

In the formula,

$R^2$ and $R^3$ are each independently a hydrocarbon group having 3 to 24 carbon atoms which contains one or more unsaturated bonds,
$R^2$ and $R^3$ are each independently a group represented by $R^{31}$-$L^2$-$R^{32}$-, or
one of $R^2$ and $R^3$ is a group represented by $R^{31}$-$L^2$-$R^{32}$-, and the other is a hydrocarbon group having 3 to 24 carbon atoms,
where $R^{31}$ represents a hydrocarbon group having 1 to 24 carbon atoms,
$L^2$ represents -O(CO)O-, -O(CO)-, -(CO)O-, -O-, or a group represented by the following structural formula, and

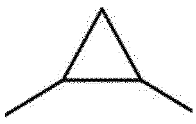

$R^{32}$ represents a divalent hydrocarbon linking group having 1 to 18 carbon atoms,
$R^5$ represents an alkyl group having 1 to 18 carbon atoms which may be substituted with -O(CO)-$R^{42}$ or -(CO)O-$R^{43}$, where $R^{42}$ and $R^{43}$ each independently represent a hydrocarbon group having 1 to 18 carbon atoms,
$R^7$ and $R^8$ each independently represent an alkyl group having 1 to 4 carbon atoms, and
e represents 2 or 3.

In Formula (2), it is preferable that one of $R^2$ and $R^3$ is a group represented by $R^{31}$-$L^2$-$R^{32}$- and the other is a hydrocarbon group having 3 to 24 carbon atoms. In Formula (2), it is preferable that $L^2$ represents -O(CO)- or -(CO)O-.

The lipid represented by Formula (1) may form a salt.

Examples of the salt in a basic group include salts with mineral acids such as hydrochloric acid, hydrobromic acid, nitric acid, and sulfuric acid; salts with organic carboxylic acids such as formic acid, acetic acid, citric acid, oxalic acid, fumaric acid, maleic acid, succinic acid, malic acid, tartaric acid, aspartic acid, trichloroacetic acid, and trifluoroacetic acid; and salts with sulfonic acids such as methanesulfonic acid, benzenesulfonic acid, p-toluenesulfonic acid, mesitylenesulfonic acid, and naphthalenesulfonic acid.

Examples of the salt in an acidic group include salts with alkali metals such as sodium and potassium; salts with alkaline earth metals such as calcium and magnesium; ammonium salts; and salts with nitrogen-containing organic bases such as trimethylamine, triethylamine, tributylamine, pyridine, N,N-dimethylaniline, N-methylpiperidine, N-methylmorpholine, diethylamine, dicyclohexylamine, procaine, dibenzylamine, N-benzyl-β-phenethylamine, 1-ephenamine, and N,N'-dibenzylethylenediamine.

Among the above salts, preferred examples of the salt include pharmacologically acceptable salts.

Preferred specific examples of the lipid represented by Formula (1) can include Compounds 1 to 10 described below.

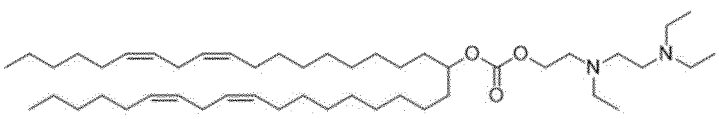

Compound 3

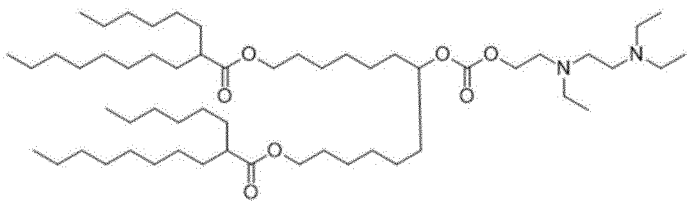

Compound 4

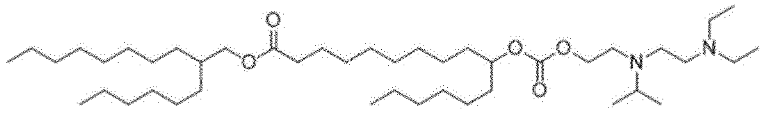

Compound 2

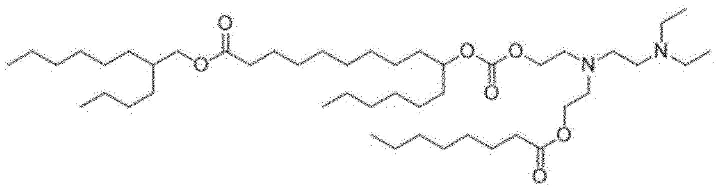

Compound 1

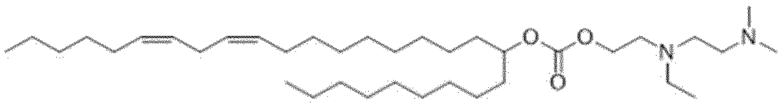

Compound 5

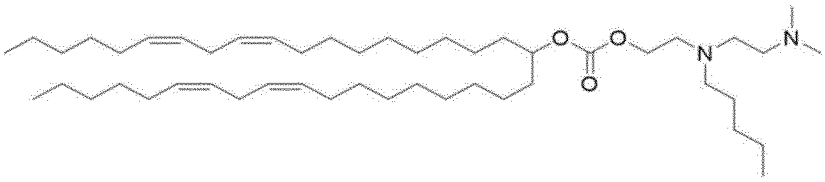

Compound 6

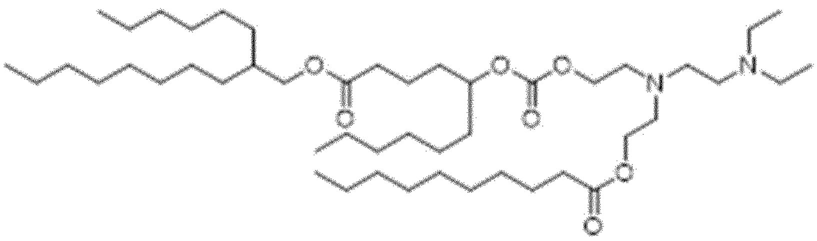

Compound 7

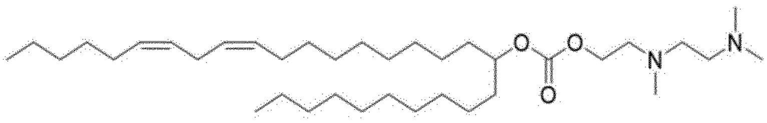

Compound 8

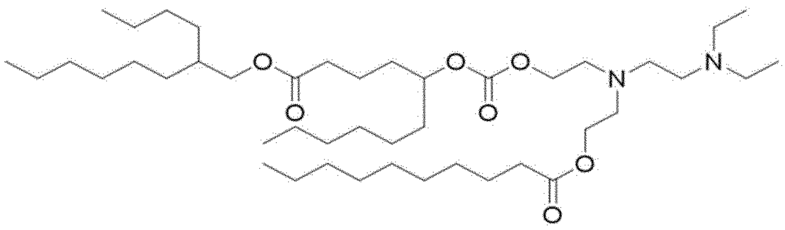

Compound 9

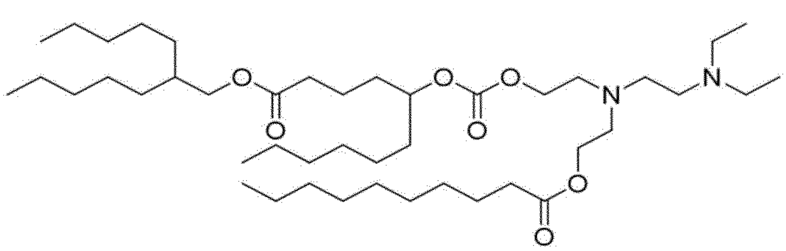

Compound 10

Among the above, Compound 1, 2, 3, 5, 6, 7, 8, 9, or 10 is preferable, and Compound 5, 6, 8, 9, or 10 is more

preferable.

The lipid represented by Formula (1) and a method for manufacturing the lipid are described in WO2019/235635A.

In the lipid composition according to the embodiment of the present invention, the content of the lipid represented by Formula (1) or a salt thereof is preferably 20 mol% to 80 mol%, more preferably 30 mol% to 70 mol%, and still more preferably 40 mol% to 60 mol%, with respect to the total amount of the lipid.

<Non-cationic lipid>

The lipid composition according to the embodiment of the present invention contains at least one non-cationic lipid.

It is preferable that the non-cationic lipid is a lipid selected from a non-ionic lipid and a zwitterionic lipid. It is more preferable that the non-cationic lipid contains at least one non-ionic lipid and at least one zwitterionic lipid.

As the non-ionic lipid, sterols are preferable. As the sterols are contained in an oil phase, the membrane fluidity can be reduced and the effect to stabilize the lipid composition can be obtained.

The sterols are not particularly limited, and examples thereof can include cholesterol, phytosterol (sitosterol, stigmasterol, fucosterol, spinasterol, brassicasterol, and the like), ergosterol, cholestanone, cholestenone, coprostanol, cholesteryl-2'-hydroxyethyl ether, and cholesteryl-4'-hydroxybutyl ether. Among these, phytosterol (sitosterol) or cholesterol is preferable. Among phytosterol (sitosterol), β-sitosterol is preferably used.

In the present invention, the content of the non-ionic lipid is preferably 10 mol% to 60 mol%, more preferably 15 mol% to 45 mol%, and still more preferably 20 mol% to 35 mol%, with respect to the total amount of the lipid.

As the zwitterionic lipid, a phospholipid is preferable, and specifically, phosphatidylcholine, phosphatidylethanolamine, or sphingomyelin is preferable. As the phospholipid, a phospholipid having a choline group such as phosphatidylcholine is preferable. The zwitterionic lipid may be used either singly or in combination of a plurality of different neutral lipids.

The phosphatidylcholine is not particularly limited, and examples thereof include soybean lecithin (SPC), hydrogenated soybean lecithin (HSPC), egg yolk lecithin (EPC), hydrogenated egg yolk lecithin (HEPC), dimyristoylphosphatidylcholine (DMPC), dipalmitoylphosphatidylcholine (DPPC), distearoylphosphatidylcholine (DSPC), dilauroylphosphatidylcholine (DLPC), and 1-palmitoyl-2-oleoylphosphatidylcholine (POPC). Among them, dimyristoylphosphatidylcholine (DMPC), distearoylphosphatidylcholine (DSPC), and dilauroylphosphatidylcholine (DLPC) are preferable, and distearoylphosphatidylcholine (DSPC) is particularly preferable.

The phosphatidylethanolamine is not particularly limited, and examples thereof include dimyristoylphosphatidylethanolamine (DMPE), dipalmitoylphosphatidylethanolamine (DPPE), distearoylphosphatidylethanolamine (DSPE), dioleoylphosphatidylethanolamine (DOPE), dilinoleoylphosphatidylethanolamine (DLoPE), diphytanoylphosphatidylethanolamine (D(Phy)PE), 1-palmitoyl-2-oleoylphosphatidylethanolamine (POPE), ditetradecylphosphatidylethanolamine, dihexadecylphosphatidylethanolamine, dioctadecylphosphatidylethanolamine and diphytanylphosphatidylethanolamine.

The sphingomyelin (SM) is not particularly limited, and examples thereof include egg yolk-derived sphingomyelin, and milk-derived sphingomyelin.

In the lipid composition according to the embodiment of the present invention, the content of the zwitterionic lipid is preferably 1 mol% or more and 30 mol% or less, more preferably 5 mol% or more and 25 mol% or less, and still more preferably 15 mol% or more and 25 mol% or less, with respect to the total amount of the lipid.

<Lipid containing hydrocarbon chain and polyethylene glycol chain>

The lipid composition according to the embodiment of the present invention contains the lipid represented by $R^{51}$-L-$(OCH_2CH_2)_n$-O-$R^{52}$ (in the formula, $R^{51}$ represents a hydrocarbon group having 6 to 30 carbon atoms, L represents -CO- or a single bond, $R^{52}$ represents a hydrogen atom or an alkyl group having 1 to 6 carbon atoms, and n represents an integer of 10 to 150) (also referred to a lipid containing a hydrocarbon chain and a polyethylene glycol chain).

$R^{51}$ preferably represents a hydrocarbon group having 8 to 26 carbon atoms, more preferably represents a hydrocarbon group having 10 to 22 carbon atoms, and still more preferably represents a hydrocarbon group having 14 to 20 carbon atoms. The hydrocarbon group represented by $R^{51}$ may be saturated or unsaturated, and may be either a linear hydrocarbon group or a branched hydrocarbon group. As the linear hydrocarbon group, a stearyl group, a palmitoyl group, or an oleyl group is particularly preferable.

L preferably represents -CO-.

$R^{52}$ preferably represents a hydrogen atom.

n represents an integer of 10 to 150, and preferably represents 15 to 80. The weight-average molecular weight of the polyethylene glycol chain represented by $-(OCH_2CH_2)_n-$ is preferably 500 to 5,000, and more preferably 750 to

3,000.

In addition, specific examples of the lipid containing the branched hydrocarbon chain and the polyethylene glycol chain are shown below.

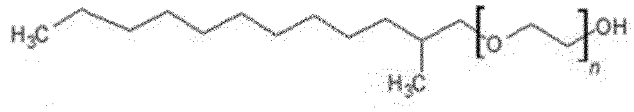

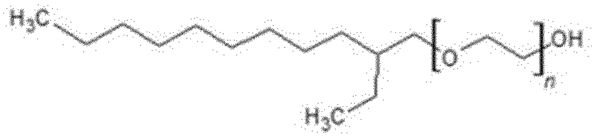

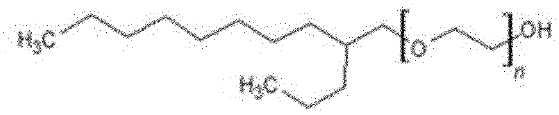

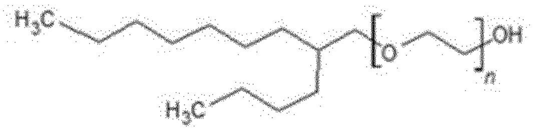

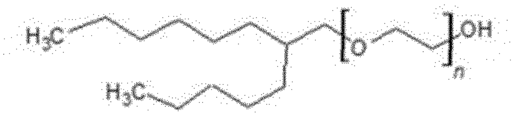

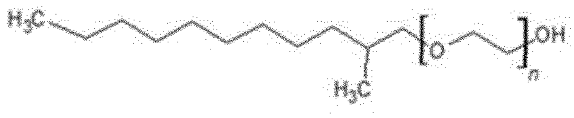

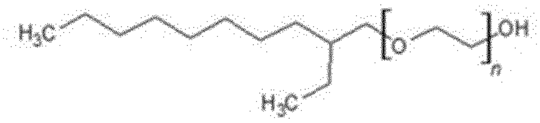

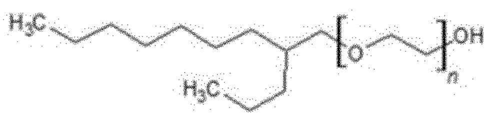

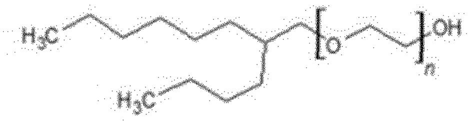

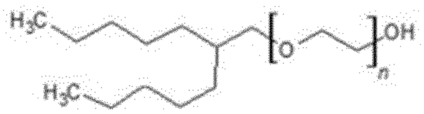

Particularly preferred specific examples of the lipid containing a hydrocarbon chain and a polyethylene glycol chain are shown below.

Polyoxyethylene stearyl ether $C_{18}H_{35}OCH_2CH_2(OCH_2CH_2)_{n-1}OH$

Polyoxyethylene isocetyl ether

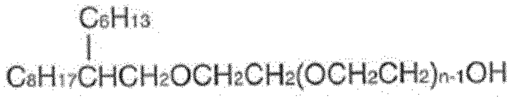

In the lipid composition according to the embodiment of the present invention, the content of the lipid containing the hydrocarbon chain and the polyethylene glycol chain is preferably 0.1 mol% to 10 mol%, more preferably 0.3 mol% to 8 mol%, and still more preferably 0.5 mol% to 5 mol%, with respect to the total amount of the lipid.

<Nucleic acid>

The lipid composition according to the embodiment of the present invention contains a nucleic acid. Examples of the nucleic acid include a plasmid, single-stranded DNA, double-stranded DNA, small interfering RNA (siRNA), micro RNA (miRNA), mRNA, an antisense oligonucleotide (as referred to ASO), and ribozyme. The lipid composition may contain any of these. In addition, the lipid composition may contain a modified nucleic acid.

In the lipid composition according to the embodiment of the present invention, in a case where the nucleic acid is an antisense oligonucleotide, the weight ratio of the lipid to the nucleic acid is preferably 5 to 100, more preferably 5 to 60, still more preferably 5 to 30, and particularly preferably 5 to 25.

<Method for manufacturing lipid composition>

The method for manufacturing the lipid composition according to the embodiment of the present invention will be described.

The method for manufacturing the lipid composition is not limited, and for example, the lipid composition can be manufactured by a method in which all of the constituent components of the lipid composition or some of oil-soluble components of the lipid composition are dissolved in an organic solvent or the like to form an oil phase, water-soluble components of the lipid composition are dissolved in water to form a water phase, and then the oil phase and the water phase are mixed together. A micromixer may be used for mixing, or an emulsification using an emulsifying machine such as a homogenizer, an ultrasonic emulsifying machine, a high-pressure injection emulsifying machine, or the like may be performed.

Alternatively, the lipid composition can also be manufactured by a method in which a lipid-containing solution is subjected to evaporation to dryness using an evaporator under reduced pressure or subjected to spray drying using a spray drier, so that a dried mixture containing a lipid is prepared, and then the mixture is added to an aqueous solvent and further emulsified using the above-described emulsifying machine or the like.

One example of the method for manufacturing the lipid composition is a method including

Step (a) of dissolving the lipid components in an organic solvent to obtain an oil phase;
Step (b) of mixing the oil phase obtained in Step (a) with a water phase containing a nucleic acid;
Step (c) of diluting the mixed solution containing the oil phase and the water phase obtained in Step (b) to obtain a dispersion liquid of lipid composition containing the nucleic acid; and
Step (d) of removing the organic solvent from the dispersion liquid of lipid composition obtained in Step (c).

Step (a) includes a process of dissolving the lipid components in an organic solvent (an alcohol such as ethanol, an ester, or the like). The total lipid concentration is not particularly limited, but is generally 1 mmol/L to 100 mmol/L, preferably 5 mmol/L to 50 mmol/L, and more preferably 10 mmol/L to 30 mmol/L.

In Step (b), the water phase can be obtained by dissolving a nucleic acid (for example, siRNA, an antisense oligonucleotide, or mRNA) in water or a buffer solution. If necessary, a component such as an antioxidant can be added. The mixing ratio (volume ratio) of water phase: oil phase is preferably 5:1 to 1:1 and more preferably 4:1 to 2:1.

In Step (c), the mixed solution can be diluted with water or a buffer solution (for example, phosphate buffered physiological saline (PBS)).

In Step (d), the method of removing the organic solvent from the dispersion liquid of lipid composition is not particularly limited, and a general method can be used. For example, the organic solvent can be removed by dialyzing the dispersion liquid with the phosphate buffered physiological saline.

The lipid composition can be subjected to sizing if necessary. The method of sizing is not particularly limited,

and the particle size can be reduced by using an extruder or the like.

<Lipid composition>

The lipid composition according to the embodiment of the present invention may be lipid particles. Lipid particles mean particles composed of a lipid, and include a composition having any structure selected from a lipid aggregate in which lipids are aggregated, a micelle, or a liposome. The structure of the lipid particles is not limited to these as long as the lipid particles are a composition containing a lipid. The liposome has a lipid bilayer structure and has a water phase in the inside, and includes a liposome which has a single bilayer membrane, and a multilamellar liposome which has multiple layers stacked together. In the present invention, any of these liposomes may be included.

The form of the lipid composition can be checked by electron microscopy, structural analysis using X-rays, or the like. For example, by a method using Cryo transmission electron microscopy (CryoTEM method), it is possible to check whether or not a lipid particle is, such as a liposome, a bimolecular lipid membrane structure (lamella structure) and a structure having an inner water layer, whether or not a lipid particle has a structure having a core with a high electron density inside the particle and packed with constituent components including a lipid, and the like. The X-ray small angle scattering (SAXS) analysis also makes it possible to check whether or not a lipid particle has a bimolecular lipid membrane structure (lamella structure).

In the case where the lipid composition according to the embodiment of the present invention is particles, the particle size of the particles is not particularly limited, but is preferably 10 to 1,000 nm, more preferably 30 to 500 nm, and still more preferably 50 to 250 nm. The particle size of the lipid particles can be measured by a general method (for example, a dynamic light scattering method, a laser diffraction method, or the like).

<Use of lipid composition>

As one example for utilizing the lipid particles in the present invention, a nucleic acid (for example, a gene) can be introduced into a cell by introducing the lipid particles containing the nucleic acid into the cell. That is, the lipid composition according to the embodiment of the present invention can be used as a pharmaceutical composition for introducing a nucleic acid into a cell. That is, the lipid composition according to the embodiment of the present invention can be used as a pharmaceutical composition for nucleic acid delivery in vivo.

In addition, in a case where the lipid particles in the present invention contains a nucleic acid for a pharmaceutical use, the lipid particles can be administered to a living body as a nucleic acid drug. In a case where the lipid composition in the present invention is used as a nucleic acid drug, the lipid composition according to the embodiment of the present invention can be administered to a living body singly or by being mixed with a pharmaceutically acceptable carrier (for example, a dosing medium such as physiological saline or a phosphate buffer solution). That is, the lipid composition according to the embodiment of the present invention may further contain a pharmaceutically acceptable carrier.

The concentration of the lipid composition (lipid particles) in the mixture with a pharmaceutically acceptable carrier is not particularly limited, but can be set to 0.05% by mass to 90% by mass in general. In addition, other pharmaceutically acceptable additives, for example, a pH adjusting buffer and an osmotic pressure adjusting agent, may be added to the nucleic acid drug containing the lipid composition according to the embodiment of the present invention.

The route of administration when the lipid composition according to the embodiment of the present invention is administered is not particularly limited, and the lipid composition can be administered by any method. Examples of the administration method include oral administration and parenteral administration (intraarticular administration, intravenous administration, intraarterial administration, subcutaneous administration, intracutaneous administration, intravitreal administration, intraperitoneal administration, intramuscular administration, intravaginal administration, intravesical administration, intrathecal administration, pulmonary administration, rectal administration, colonic administration, buccal administration, nasal administration, intracisternal administration, inhalation, and the like). Parenteral administration is preferable. As the administration method, intravenous injection, subcutaneous injection, intracutaneous injection, or intramuscular injection is preferable. As for the administration, it is preferable to deliver the nucleic acid by topical administration in vivo. The lipid composition according to the embodiment of the present invention can also be administered by being directly injected into the affected area. In addition, the area to be administrated in the case of performing topical administration in vivo is not particularly limited, and one example is administration to the central nervous system.

The dosage form of the lipid composition according to the embodiment of the present invention is not particularly limited. In a case of performing oral administration, the lipid composition according to the embodiment of the present invention can be used in the form of tablets, troches, capsules, pills, suspension, syrup, or the like by being combined with an appropriate excipient. In addition, pharmaceutical preparation suitable for parenteral administration can appropriately contain an antioxidant, a buffer, a bacteriostat, and additives such as an isotonic sterile injection, a suspending agent, a solubilizer, a thickener, a stabilizer, or a preservative.

<Nucleic acid delivery carrier>

The lipid composition according to the embodiment of the present invention can retain a nucleic acid at a high encapsulation rate and thereby is extremely useful as a nucleic acid delivery carrier. According to the nucleic acid delivery carrier using the present invention, the nucleic acid and the like can be introduced into the cells, for example, by mixing the obtained lipid composition with the nucleic acid and the like and transfecting the mixture in vitro or in vivo. In addition, the nucleic acid delivery carrier using the present invention is also useful as a nucleic acid delivery carrier in nucleic acid drugs. That is, the lipid composition according to the embodiment of the present invention is useful as a composition for nucleic acid delivery in vitro or in vivo (preferably in vivo).
Next, the present invention will be described based on examples, but the present invention is not limited thereto.

Examples

(PTEN antisense oligonucleotide information)

Phosphatase and Tensin Homolog Deleted from Chromosome 10 (PTEN) is an enzyme that catalyzes a dephosphorylation reaction of phosphatidylinositol 3,4,5-triphosphate, which is an inositol phospholipid.
An antisense oligonucleotide nucleic acid (PTEN ASO) for a PTEN protein was purchased from Hokkaido System Science Co., Ltd. PTEN ASO is an oligonucleotide consisting of 20 bases, in which nucleotides are connected by a phosphodiester bond, and the sequence of which is described below.
5(m)^t(m)^g(m)^5(m)^t(m)^a^g^5c^5c^t^5c^t^g^g^a^t(m)^t(m)^t(m)^g(m)^a(m)
Here, small letters (a, g, and t) represent adenin, guanine, and thymidin, respectively, and (m) represents 2'-MOE modification.
5(m) represents 2'-MOE 5-Me cytosine, t(m) represents 2'-MOE thymidin, g(m) represents 2'-MOE guanine, a(m) represents 2'-MOE adenine, 5c represents 5-methyl-cytosine and ^ represents phosphorothioate.
2'-MOE represents 2'-O-methoxyethyl.

(Preparation of PTEN ASO-LNP)

The first lipid, phospholipid, cholesterol, and polyethylene glycol lipid (PEG lipid) shown in Table 1 were dissolved in ethanol at the molar ratio shown in Table 1 such that the total lipid concentration was 20 mmol/L to obtain the oil phase.

The components used are shown below.

1,2-Distearoyl-sn-glycero-3-phosphocholine (DSPC, product name: COATSOME MC-8080, NOF corporation)
1,2-Dimyristoyl-sn-glycero-3-phosphocholine (DMPC, product name: COATSOME MC-4040, NOF corporation)
1,2-Dilauroyl-sn-glycero-3-phosphocholine (DLPC, product name: 12:0 PC (DLPC), Avanti polar lipids)
Egg sphingomyelin (SM, product name: COATSOME NM-10, NOF corporation) Cholesterol (Product name: Cholesterol HP, Nippon Seika Co., Ltd.)
Monostearyl PEG (product name: Polyethylene Glycol Monostearate (4E.O.), FUJIFILM Wako Pure Chemical Corporation)
1,2-Dimyristoyl-rac-glycero-3-methoxypolyethyleneglycol (DMG-PEG2000, product name: SUNBRIGHT (register trademark) GM-020, NOF corporation)
The structure of Monostaryl PEG (also referred to mono PEG) is shown below.

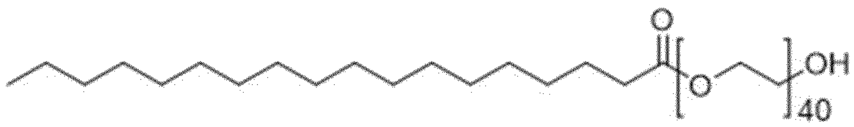

The structure of DMG-PEG2000 (also referred to DMG-PEG) is shown below.

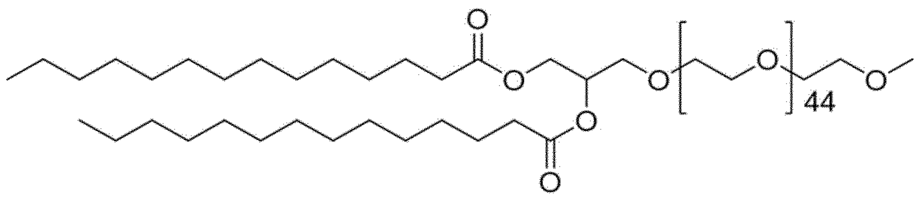

The structures of Dlin-MC3-DMA, DOTMA, and DODAP are shown below.

D l i n－MC 3－DMA

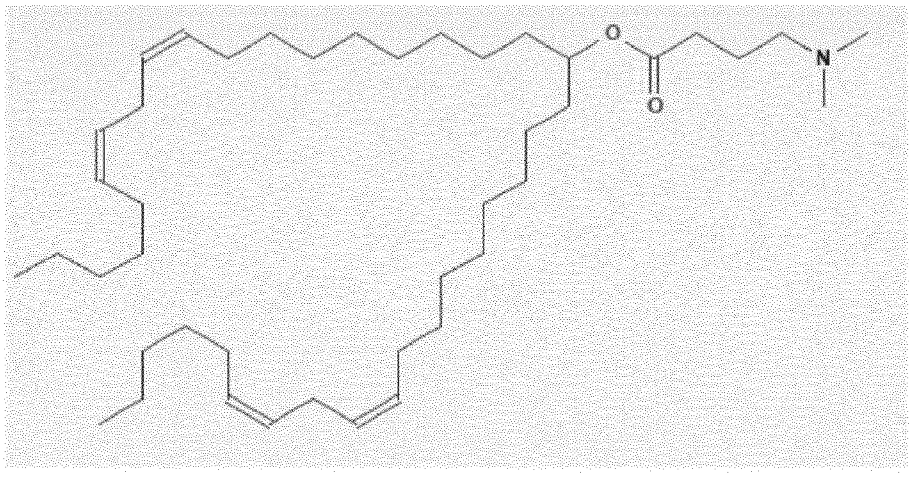

DOTMA : 1,2-di-O-octadecenyl-3-trimethylammoniumpropane (chloride salt)

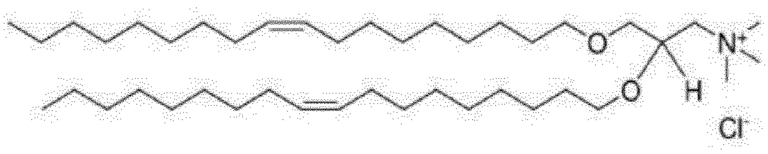

D O D A Y : 1,2-dioleoyloxy-3-dimethylaminopropane

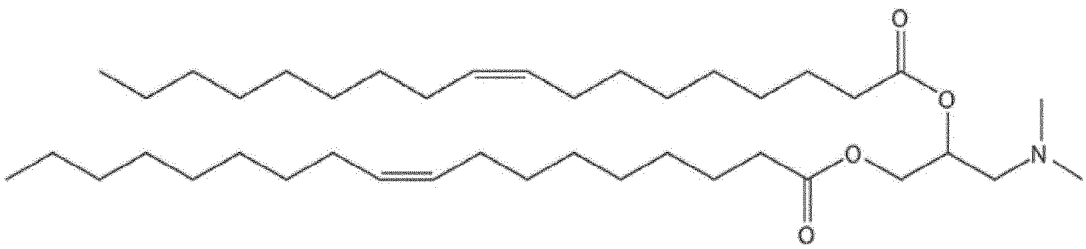

5 mg of PTEN ASO was dissolved in 1 mL of sterile water and diluted with a 10 mmol/L acetate buffer having a pH of 4 such that the nucleic acid concentration was 54.6 μmol/L, to obtain a water phase. Then the water phase and the oil phase were mixed together with a micromixer (see JP5288254B) using a syringe pump such that the volume ratio of water phase:oil phase was 3:1, and the mixed solution was two-fold diluted with a phosphate buffered physiological saline (PBS), thereby obtaining a nucleic acid lipid particle dispersion.

The molar ratios of the first lipid, the phospholipid, the sterol, and the PEG lipid in the lipid composition are shown in Tables 1 and 2.

The mass ratio of the nucleic acid to the total lipids at the time of mixing is also shown in Tables 1 and 2.

[Table 1]

| | First lipid | Phospholipid | PEG lipid | Composition ratio of lipids (mol%) | | | | Mass ratio of nucleic acid to total lipids |
|---|---|---|---|---|---|---|---|---|
| | | | | First lipid | Phospholipid | Sterol | PEG lipid | |
| | | | | | | Cholesterol | | |
| Example 1 | Compound 1 | DSPC | monostearyl PEG | 50 | 20 | 28.5 | 1.5 | 11.8 |
| Example 2 | Compound 2 | DSPC | monostearyl PEG | 50 | 20 | 28.5 | 1.5 | 11.8 |
| Example 3 | Compound 1 | DMPC | monostearyl PEG | 50 | 20 | 28.5 | 1.5 | 11.8 |
| Example 4 | Compound 1 | DLPC | monostearyl PEG | 50 | 20 | 28.5 | 1.5 | 11.8 |
| Example 5 | Compound 1 | SM | monostearyl PEG | 50 | 20 | 28.5 | 1.5 | 11.8 |
| Example 6 | Compound 1 | DSPC | monostearyl PEG | 50 | 20 | 27 | 3 | 11.8 |
| Example 7 | Compound 1 | DSPC | monostearyl PEG | 50 | 20 | 25.5 | 4.5 | 11.8 |
| Example 8 | Compound 1 | DMPC | monostearyl PEG | 50 | 20 | 28.5 | 1.5 | 20.2 |
| Example 9 | Compound 1 | DLPC | monostearyl PEG | 50 | 20 | 28.5 | 1.5 | 51.7 |
| Example 10 | Compound 5 | DSPC | monostearyl PEG | 50 | 20 | 28.5 | 1.5 | 12.7 |
| Example 11 | Compound 6 | DSPC | monostearyl PEG | 50 | 20 | 28.5 | 1.5 | 12.7 |
| Example 12 | Compound 3 | DSPC | monostearyl PEG | 50 | 20 | 28.5 | 1.5 | 12.7 |
| Example 13 | Compound 7 | DSPC | monostearyl PEG | 50 | 20 | 28.5 | 1.5 | 12.7 |
| Example 14 | Compound 8 | DSPC | monostearyl PEG | 50 | 20 | 28.5 | 1.5 | 12.7 |
| Example 15 | Compound 9 | DSPC | monostearyl PEG | 50 | 20 | 28.5 | 1.5 | 12.7 |
| Example 16 | Compound 10 | DSPC | monostearyl PEG | 50 | 20 | 28.5 | 1.5 | 12.7 |
| Comparative Example 1 | Compound 1 | DSPC | DMG-PEG | 50 | 20 | 28.5 | 1.5 | 11.8 |
| Comparative Example 2 | Dlin-MC3-DM A | DSPC | DMG-PEG | 50 | 20 | 28.5 | 1.5 | 11.8 |
| Comparative Example 3 | DOTMA | DSPC | DMG-PEG | 50 | 20 | 28.5 | 1.5 | 11.8 |
| Comparative Example 4 | DODAP | DSPC | DMG-PEG | 50 | 20 | 28.5 | 1.5 | 11.8 |
| Comparative Example 5 | Dlin-MC3-DM A | DSPC | monostearyl PEG | 50 | 20 | 28.5 | 1.5 | 11.8 |
| Comparative Example 6 | DOTMA | DSPC | monostearyl PEG | 50 | 20 | 28.5 | 1.5 | 11.8 |
| Comparative Example 7 | DODAP | DSPC | monostearyl PEG | 50 | 20 | 28.5 | 1.5 | 11.8 |

[Table 2]

| | First lipid | Phospholipid | PEG lipid | Composition ratio of lipids (mol%) | | | | Mass ratio of nucleic acid to total lipids |
|---|---|---|---|---|---|---|---|---|
| | | | | First lipid | Phospholipid | Sterol: β-Sitosterol | PEG lipid | |
| Example 17 | Compound 10 | DSPC | monostearyl PEG | 50 | 10 | 38.5 | 1.5 | 12.7 |

<Measurement of particle size>

The particle size and polydispersion index of the lipid particles were measured by 10-fold diluting the dispersion liquid of lipid particle with phosphate buffered physiological saline (PBS) using Zeta-potential & Particle size Analyzer ELS-Z2 (Otsuka Electronics Co., Ltd.). The measurement results are shown in Table 3.

<Evaluation of encapsulation rate of PTEN ASO>

(Quantification of total nucleic acid concentration)

30 μL of 3 mol/L aqueous sodium acetate solution and 9 μL of glycogen were added to 60 μL of the lipid particles retaining nucleic acids, and then 1.5 mL of ethanol was added thereto, so that only the nucleic acids were precipitated while the lipid was dissolved. Then, the supernatant was removed by centrifugation. The precipitates were air-dried for 15 minutes or longer, then water was added thereto to redissolve the precipitates, and the concentration thereof was measured using Nanodrop ND1000 (Thermo Fischer Scientific), thereby quantifying the total nucleic acid concentration.

(Quantification of nucleic acid concentration in outer water phase)

The nucleic acid concentration was quantified using a Quant-iT RiboGreen RNA Assay Kit (Thermo Fischer Scientific) according to the protocol. First, a 20× TE buffer included in the above kit was diluted with water, thereby obtaining a 1× TE buffer. TE represents Tris/EDTA (ethylenediaminetetraacetic acid). In order to quantify only the nucleic acid in the outer water phase, the dispersion liquid of lipid particles retaining nucleic acids was 10,000-fold diluted with the 1× TE buffer.

100 μL of the 10,000-fold diluted dispersion liquid of lipid particles was put in a 96-well plate, then 100 μL of a RiboGreen reagent (reagent included in the Quanti-iT Ribogreen RNA Assay Kit described above) 2,000-fold diluted with the 1× TE buffer was added to a sample, and fluorescence (excitation wavelength: 485 nm, fluorescence wavelength: 535 nm) was measured using a plate reader Infinite F200 (TECAN), thereby quantifying the nucleic acid concentration in the outer water phase.

(Calculation of encapsulation rate)

By using the quantification results of the total nucleic acid concentration and the nucleic acid concentration in the outer water phase that were obtained through the above steps, the nucleic acid encapsulation rate of the nucleic acid lipid particles was calculated according to the following Equation.

$$\text{Nucleic acid encapsulation rate (\%)} = (\text{total nucleic acid concentration} - \text{nucleic acid concentration in outer water phase})/\text{total nucleic acid concentration} \times 100$$

The calculation results are shown in Table 3.

[Table 3]

| | Cumulant particle size (nm) | Polydispersion index | Encapsulation rate (%) |
|---|---|---|---|
| Example 1 | 77.7 | 0.087 | 90% |
| Example 2 | 83.6 | 0.166 | 86% |
| Example 3 | 85 | 0.175 | 86% |
| Example 4 | 85.7 | 0.164 | 93% |
| Example 5 | 96.5 | 0.174 | 68% |
| Example 6 | 68.7 | 0.192 | 60% |
| Example 7 | 62.6 | 0.191 | 51% |
| Example 8 | 64.6 | 0.179 | 85% |
| Example 9 | 64.2 | 0.166 | 86% |
| Example 10 | 72.1 | 0.049 | 98% |
| Example 11 | 60.4 | 0.067 | 98% |
| Example 12 | 58.6 | 0.041 | 98% |
| Example 13 | 81.6 | 0.040 | 98% |
| Example 14 | 92.7 | 0.038 | >98% |
| Example 15 | 76.4 | 0.044 | >98% |
| Example 16 | 72.1 | 0.049 | 98% |
| Example 17 | 96.6 | 0.082 | 62% |
| Comparative Example 1 | 61.4 | 0.188 | 71% |
| Comparative Example 2 | 75.4 | 0.122 | 76% |
| Comparative Example 3 | 100.2 | 0.137 | 89% |
| Comparative Example 4 | 69.1 | 0.077 | 90% |
| Comparative Example 5 | 104.5 | 0.109 | 92% |
| Comparative Example 6 | 260.4 | 0.146 | 82% |
| Comparative Example 7 | 98.5 | 0.023 | 96% |

<Evaluation of PTEN mRNA knockdown in vitro>

(Cells used for evaluation)

In an in vitro test using A431 cells (American Type Culture Collection), E-MEM (gibco), fetal bovine serum (FBS, gibco), Penicillin-streptomycin (gibco), and non-essential amino acid (NEAA, FUJIFII,M Wako Pure Chemical Corporation) were mixed at a ratio of 88:10:1:1 and the mixture was used as a culture medium.

In an in vitro test using SH-SY5Y cells (American Type Culture Collection), E-MEM (gibco), Ham's F12 (gibco), FBS (gibco), Penicillin-streptomycin (gibco), and NEAA (FUJIFILM Wako Pure Chemical Corporation) were mixed at a ratio of 41.5:41.5:15:1:1 and the mixture was used as a culture medium.

(Quantification of PTEN mRNA by PCR)

The measurement of PTEN protein mRNA was performed according to the protocol of TaqMan (register trademark) Fast Advanced Cells-to-CT™ Kit (Thermo Fischer Scientific). To A431 cells or SH-SY5Y cells, a dispersion liquid of nucleic acid lipid particles prepared to have a final concentration of 500 nmol/L as an ASO concentration, Naked ASO or PBS was added. After exposure for 24 hours under the control of 37°C and 5% $CO_2$, the culture supernatant was removed, and the mixture was washed once with PBS at 4°C. After removing the PBS, a lysis solution was added at 50 μL/well, and the mixture was stood still for 5 minutes at room temperature to obtain a cell lysate. For the cell lysate,

TaqMan (register trademark) Fast Advanced Cells-to-CT™ Kit (Thermo Fischer scientific), and Hs02621230_s1, FAM/MGB (Thermo Fischer Scientific) and Human GAPDH Endogenous Control (VIC (register trademark)/MGB, Thermo Fischer Scientific) as a PCR reaction reagent were used to perform reverse transcription and PCR reaction.

The PTEN mRNA level of each sample was calculated by the ΔΔCt method. Specifically, the Ct value of GAPDH is subtracted from the Ct value of PTEN to calculate the ΔCt value of each sample. The average value of the ΔCt values of the PBS treatment group was subtracted from the calculated ΔCt value to calculate the ΔΔCt value. For each ΔΔCt value, a relative value with respect to the Naked ASO to be compared was taken as a PTEN mRNA relative value.

Fig. 1 shows the results of comparison in a case where the type of the cationic lipid was changed (Examples 1 and Comparative Examples 1 to 7). Fig. 1 shows the test results in case of using A431 cells. A synergistic effect was confirmed in a case where the lipid represented by Formula (1) was used.

Fig. 2 shows the results of comparison in a case where the type of the phospholipid was changed (Examples 1 and 3 to 5). Fig. 2 shows the test results in case of using A431 cells. High drug efficacy was confirmed in all of Examples 1 and 3 to 5.

Fig. 3 shows the results of comparison in a case where the mol% of the PEG lipid was changed (Examples 1, 6, and 7). Fig. 3 shows the test results in case of using SH-SY5Y cells. High drug efficacy was confirmed in all of Examples 1, 6, and 7.

Fig. 4 shows the results of comparison in a case where the lipid/RNA ratio was changed (Examples 1, 8, and 9). Fig. 4 shows the test results in case of using SH-SY5Y cells. High drug efficacy was confirmed in all of Examples 1, 8, and 9.

Furthermore, Table 4 shows the results of changing the type of the cationic lipid contained in Formula (1) (Examples 10 to 16) and the results of changing the sterol (Example 17). Table 4 shows test results in case of using A431 cells and SH-SY5Y cells. High drug efficacy was confirmed in all of Examples 10 to 17.

[Table 4]

| | A431 cell | SH-SY5Y cell |
|---|---|---|
| Example 10 | 0.87 | 0.34 |
| Example 11 | 0.12 | 0.81 |
| Example 12 | 0.17 | 1.22 |
| Example 13 | 1.13 | 0.37 |
| Example 14 | 0.86 | 0.36 |
| Example 15 | 1.12 | 0.30 |
| Example 16 | 1.04 | 0.14 |
| Example 17 | 0.03 | 0.04 |

<Evaluation of PTEN mRNA knockdown in intracerebroventricular administration>

(Administration to sample preparation)

Crl:CD(SD) rats (Charles River Laboratories Japan, Inc.) were allocated to groups (n = 8) based on a body-weight stratified randomization. The dispersion liquid of the nucleic acid lipid particles of Example 1, the dispersion liquid of the nucleic acid lipid particles of Example 15, or 10 μL of Naked ASO was administered intracerebroventricularly at an ASO amount of 100 μg/Head. In addition, the same volume of a 10% sucrose solution was administered intracerebroventricularly as a control (medium group).

14 days after administration, the rats were euthanized under anesthesia to collect the cerebral cortex. The obtained cerebral cortex was immersed in an RNAlater™ Stabilization Solution (Thermo Fischer Scientific). Buffer RLT plus (RNeasy plus mini kit, QIAGEN) was added thereto, and the tissue was disrupted using a biomasher, and then the supernatant was obtained by centrifugation (4°C, 10,000 g, 20 minutes). The supernatant was purified using a spin column (QIA shredder, QIAGEN), 70% ethanol was added thereto, and mRNA was extracted using an RNeasy spin column. The RNA concentration of the mRNA extract solution was measured with Nanodrop ND-1000 (Thermo Fischer Scientific).

(Quantification of PTEN mRNA by PCR)

Each sample was diluted with RNase free water to ensure that the RNA concentrations were equal, and then a reverse transcription reaction (37°C., 30 minutes) was performed using a High-Capacity RNA-to-cDNA™ Kit (Thermo Fischer Scientific). A PCR reaction of the reverse transcription sample was performed using TaqMan Gene Expression Master Mix (Thermo Fischer Scientific), Rn00477208_m1, VIC/MGB_PL (Thermo Fischer Scientific), Rn01775763_g1, and FAM/MGB (Thermo Fischer Scientific) as reaction reagents.

The PTEN mRNA levels of each nucleic acid lipid particle group and the Naked ASO group were calculated by the $\Delta\Delta$Ct method. Specifically, the Ct value of GAPDH is subtracted from the Ct value of PTEN to calculate the $\Delta$Ct value of each sample. The average value of the $\Delta$Ct values of the medium group was subtracted from the calculated $\Delta$Ct value to calculate the $\Delta\Delta$Ct value, and the KD rate of PTEN mRNA of each sample was calculated based on the calculated $\Delta\Delta$Ct. Fig. 5 shows the results of Example 1 and Naked ASO, and Fig. 6 shows the results of Example 15 and Naked ASO. The nucleic acid lipid particles in the present invention exhibited high drug efficacy as compared with Naked ASO (Comparative Example).

## Claims

**1.** A lipid composition comprising:

a lipid represented by Formula (1) or a salt thereof;
a nucleic acid;
at least one non-cationic lipid; and
a lipid represented by $R^{51}$-L-$(OCH_2CH_2)_n$-O-$R^{52}$ (in the formula, $R^{51}$ represents a hydrocarbon group having 6 to 30 carbon atoms, L represents -CO- or a single bond, $R^{52}$ represents a hydrogen atom or an alkyl group having 1 to 6 carbon atoms, and n represents an integer of 10 to 150),

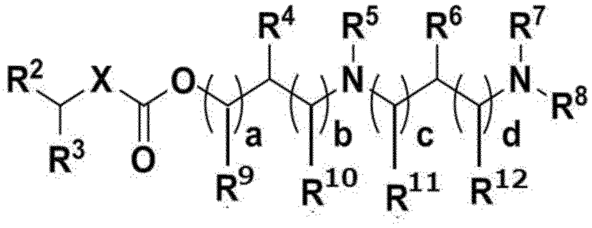

(1)

in the formula, X represents $-NR^1-$ or -O-,
$R^1$ represents a hydrogen atom, a hydrocarbon group having 6 to 24 carbon atoms, or a group represented by $R^{21}$-$L^1$-$R^{22}$-, where $R^{21}$ represents a hydrocarbon group having 1 to 24 carbon atoms, $L^1$ represents -O(CO)O-, -O(CO)-, -(CO)O-, -O-, or a group represented by the following structural formula, and

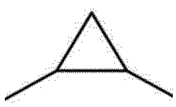

$R^{22}$ represents a divalent hydrocarbon linking group having 1 to 18 carbon atoms,
$R^2$ and $R^3$ each independently represent a hydrogen atom, a hydrocarbon group having 3 to 24 carbon atoms, or a group represented by $R^{31}$-$L^2$-$R^{32}$-, where $R^{31}$ represents a hydrocarbon group having 1 to 24 carbon atoms, $L^2$ represents -O(CO)O-, -O(CO)-, -(CO)O-, -O-, or a group represented by the following structural formula, and

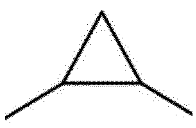

$R^{32}$ represents a divalent hydrocarbon linking group having 1 to 18 carbon atoms,
$R^4$, $R^5$, $R^6$, $R^7$, $R^8$, $R^9$, $R^{10}$, $R^{11}$, and $R^{12}$ each independently represent a hydrogen atom or an alkyl group having 1 to 18 carbon atoms which may be substituted,
groups in any one or more pairs among $R^4$ and $R^5$, $R^{10}$ and $R^5$, $R^5$ and $R^{12}$, $R^4$ and $R^6$, $R^5$ and $R^6$, $R^6$ and $R^7$, $R^6$ and $R^{10}$, $R^{12}$ and $R^7$, and $R^7$ and $R^8$ may be linked to each other to form a 4- to 7-membered ring which may contain an O atom,
a substituent on the alkyl group having 1 to 18 carbon atoms which may be substituted is a hydroxyl group, a carboxyl group, an amino group represented by $-NR^{45}R^{46}$, a substituted or unsubstituted aryl group, a substituted or unsubstituted heteroaryl group, or a group represented by $-O(CO)O-R^{41}$, $-O(CO)-R^{42}$, $-(CO)O-R^{43}$, or $-O-R^{44}$, where $R^{41}$, $R^{42}$, $R^{43}$, $R^{44}$, $R^{45}$ and $R^{46}$ each independently represent a hydrocarbon group having 1 to 18 carbon atoms,
a substituent on the substituted or unsubstituted aryl group and on the substituted or unsubstituted heteroaryl group is an alkyl group having 1 to 18 carbon atoms, a hydroxyl group, a carboxyl group, an amino group represented by $-NR^{45}R^{46}$, or a group represented by $-O(CO)O-R^{41}$, $-O(CO)-R^{42}$, $-(CO)O-R^{43}$, or $-O-R^{44}$, where $R^{41}$, $R^{42}$, $R^{43}$, $R^{44}$, $R^{45}$, and $R^{46}$ each independently represent a hydrocarbon group having 1 to 18 carbon atoms, and
a, b, c, and d each independently represent an integer of 0 to 3, provided that a + b is 1 or more, and c + d is 1 or more.

**2.** The lipid composition according to claim 1, wherein the lipid represented by Formula (1) is a lipid represented by Formula (2),

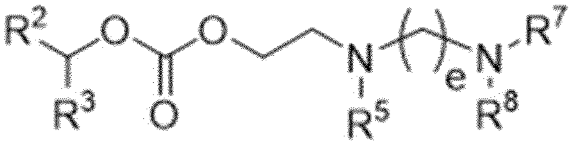
(2)

in the formula,

$R^2$ and $R^3$ are each independently a hydrocarbon group having 3 to 24 carbon atoms which contains one or more unsaturated bonds,
$R^2$ and $R^3$ are each independently a group represented by $R^{31}-L^2-R^{32}-$, or
one of $R^2$ and $R^3$ is a group represented by $R^{31}-L^2-R^{32}-$, and the other is a hydrocarbon group having 3 to 24 carbon atoms,
where $R^{31}$ represents a hydrocarbon group having 1 to 24 carbon atoms,
$L^2$ represents -O(CO)O-, -O(CO)-, -(CO)O-, -O-, or a group represented by the following structural formula, and

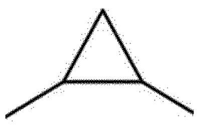

$R^{32}$ represents a divalent hydrocarbon linking group having 1 to 18 carbon atoms,
$R^5$ represents an alkyl group having 1 to 18 carbon atoms which may be substituted with $-O(CO)-R^{42}$ or $-(CO)O-R^{43}$, where $R^{42}$ and $R^{43}$ each independently represent a hydrocarbon group having 1 to 18 carbon atoms,
$R^7$ and $R^8$ each independently represent an alkyl group having 1 to 4 carbon atoms, and
e represents 2 or 3.

**3.** The lipid composition according to claim 1 or 2,
wherein a content of the lipid represented by Formula (1) is 30 to 70 mol% with respect to a total amount of the lipid.

**4.** The lipid composition according to any one of claims 1 to 3,
wherein the non-cationic lipid is a lipid selected from a non-ionic lipid and a zwitterionic lipid.

**5.** The lipid composition according to claim 4,
wherein the non-cationic lipid includes at least one non-ionic lipid and at least one zwitterionic lipid.

**6.** The lipid composition according to claim 4 or 5, wherein the non-ionic lipid is sterols.

**7.** The lipid composition according to claim 6, wherein the sterols are phytosterol or cholesterol.

**8.** The lipid composition according to any one of claims 5 to 7, wherein a content of the non-ionic lipid is 10 to 60 mol% with respect to a total amount of the lipid.

**9.** The lipid composition according to claim 4 or 5, wherein the zwitterionic lipid is a phospholipid.

**10.** The lipid composition according to any one of claims 4 to 9, wherein a content of the zwitterionic lipid is 1 to 30 mol% with respect to a total amount of the lipid.

**11.** The lipid composition according to any one of claims 1 to 10, wherein $R^{51}$ represents a hydrocarbon group having 8 to 26 carbon atoms.

**12.** The lipid composition according to any one of claims 1 to 11, wherein a content of the lipid represented by $R^{51}$-L-$(OCH_2CH_2)_n$-O-$R^{52}$ is 0.1 to 10 mol% with respect to a total amount of the lipid.

**13.** The lipid composition according to any one of claims 1 to 12, wherein a weight ratio of the lipid to the nucleic acid is 5 to 100.

**14.** The lipid composition according to any one of claims 1 to 13, further comprising a pharmaceutically acceptable carrier.

**15.** The lipid composition according to claim 14, wherein the lipid composition is a pharmaceutical composition for introducing a nucleic acid into a cell.

**16.** The lipid composition according to claim 15, wherein the lipid composition is a pharmaceutical composition for nucleic acid delivery in vivo.

**17.** The lipid composition according to claim 16, wherein the lipid composition is a pharmaceutical composition for nucleic acid delivery in vivo by topical administration.

**18.** The lipid composition according to claim 17, wherein the topical administration in vivo is administration to a central nervous system.

**19.** The lipid composition according to any one of claims 1 to 13, wherein the lipid composition is a nucleic acid delivery carrier.

FIG. 1

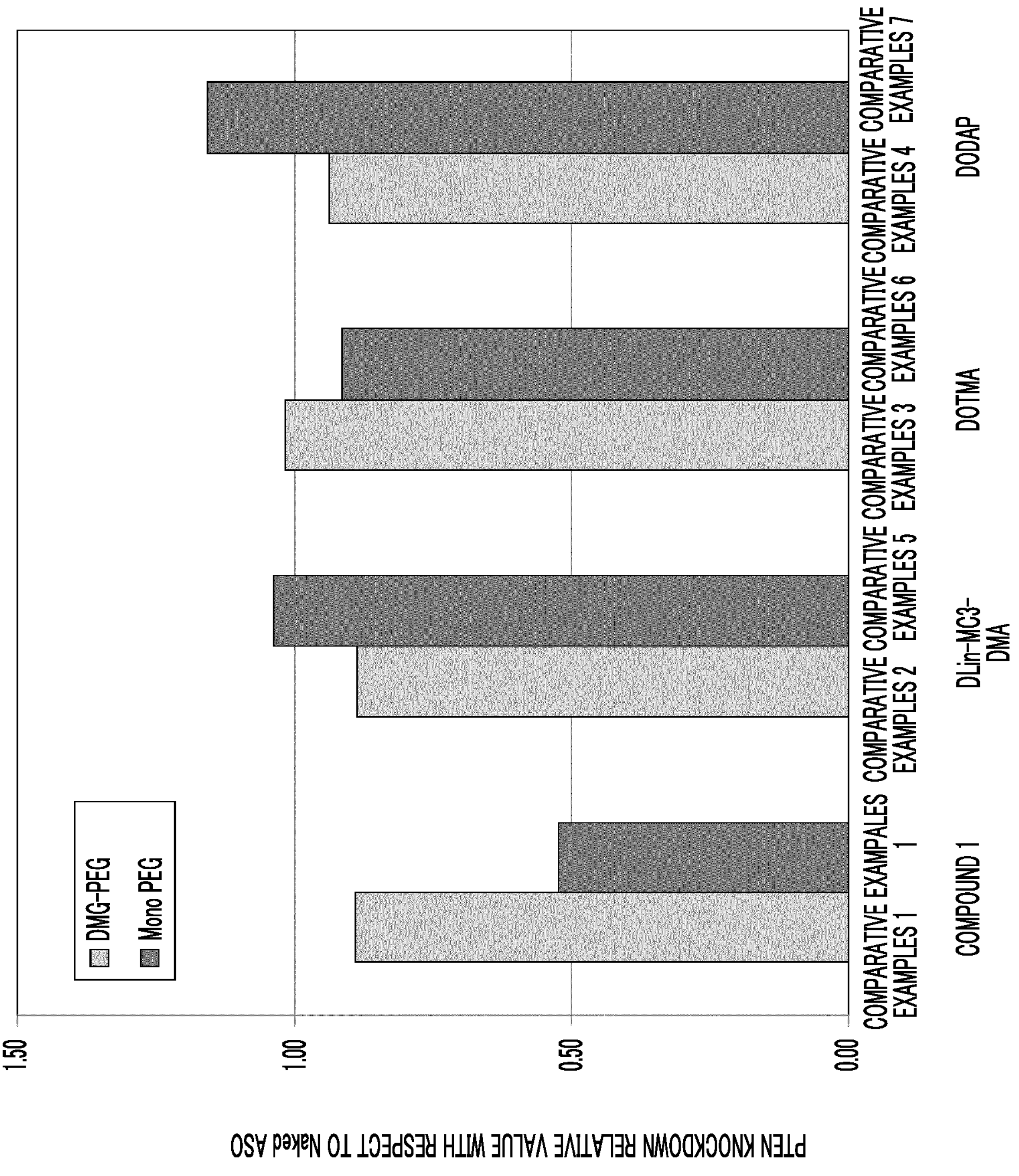
PTEN KNOCKDOWN RELATIVE VALUE WITH RESPECT TO Naked ASO
0.00
0.50
1.00
1.50
DMG-PEG
Mono PEG
COMPARATIVE EXAMPLES 1
COMPARATIVE EXAMPALES 1
COMPOUND 1
COMPARATIVE EXAMPLES 2
COMPARATIVE EXAMPLES 5
DLin-MC3-DMA
COMPARATIVE EXAMPLES 3
COMPARATIVE EXAMPLES 6
DOTMA
COMPARATIVE EXAMPLES 4
COMPARATIVE EXAMPLES 7
DODAP

FIG. 2

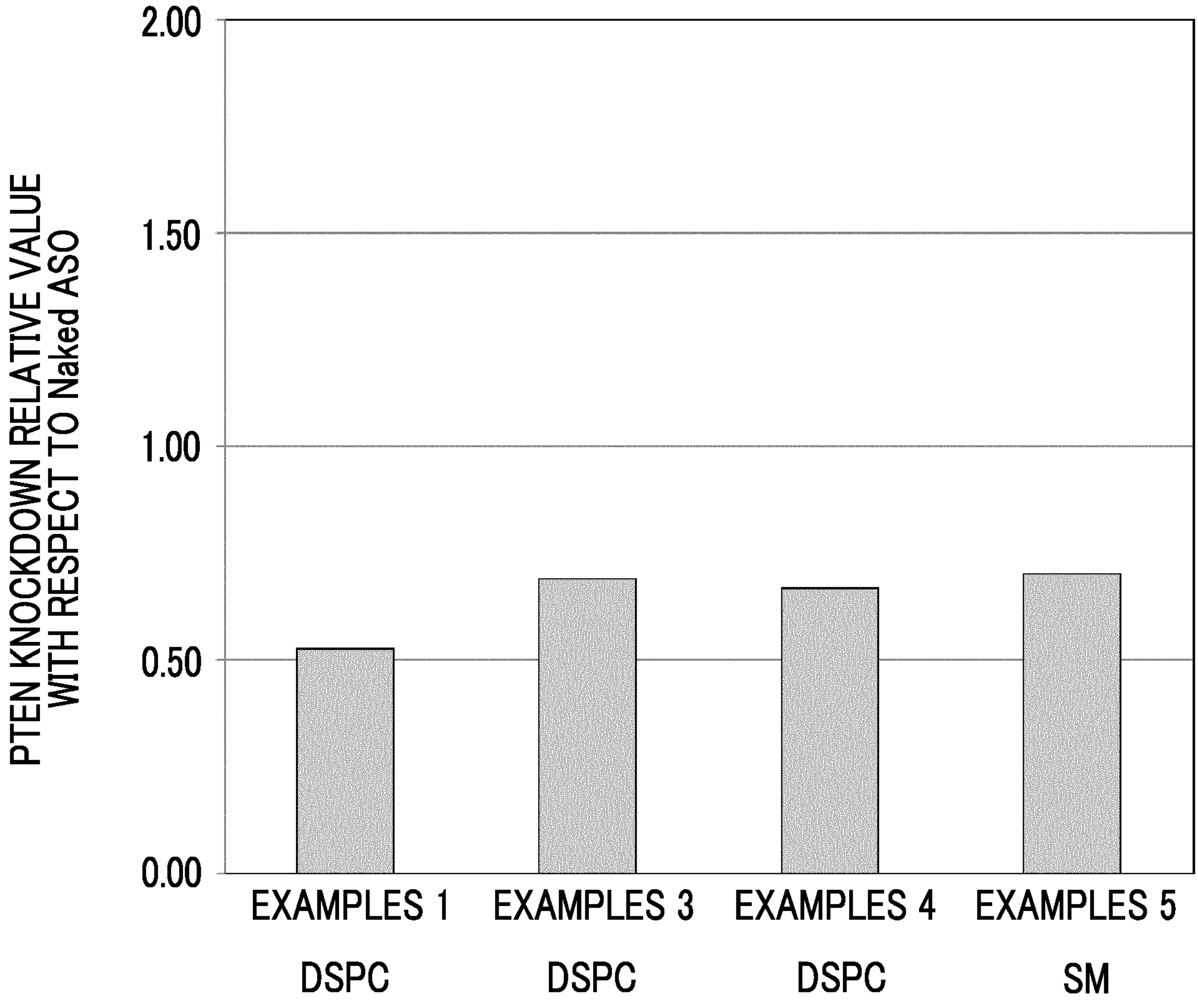
PTEN KNOCKDOWN RELATIVE VALUE WITH RESPECT TO Naked ASO
2.00
1.50
1.00
0.50
0.00
EXAMPLES 1
DSPC
EXAMPLES 3
DSPC
EXAMPLES 4
DSPC
EXAMPLES 5
SM

FIG. 3

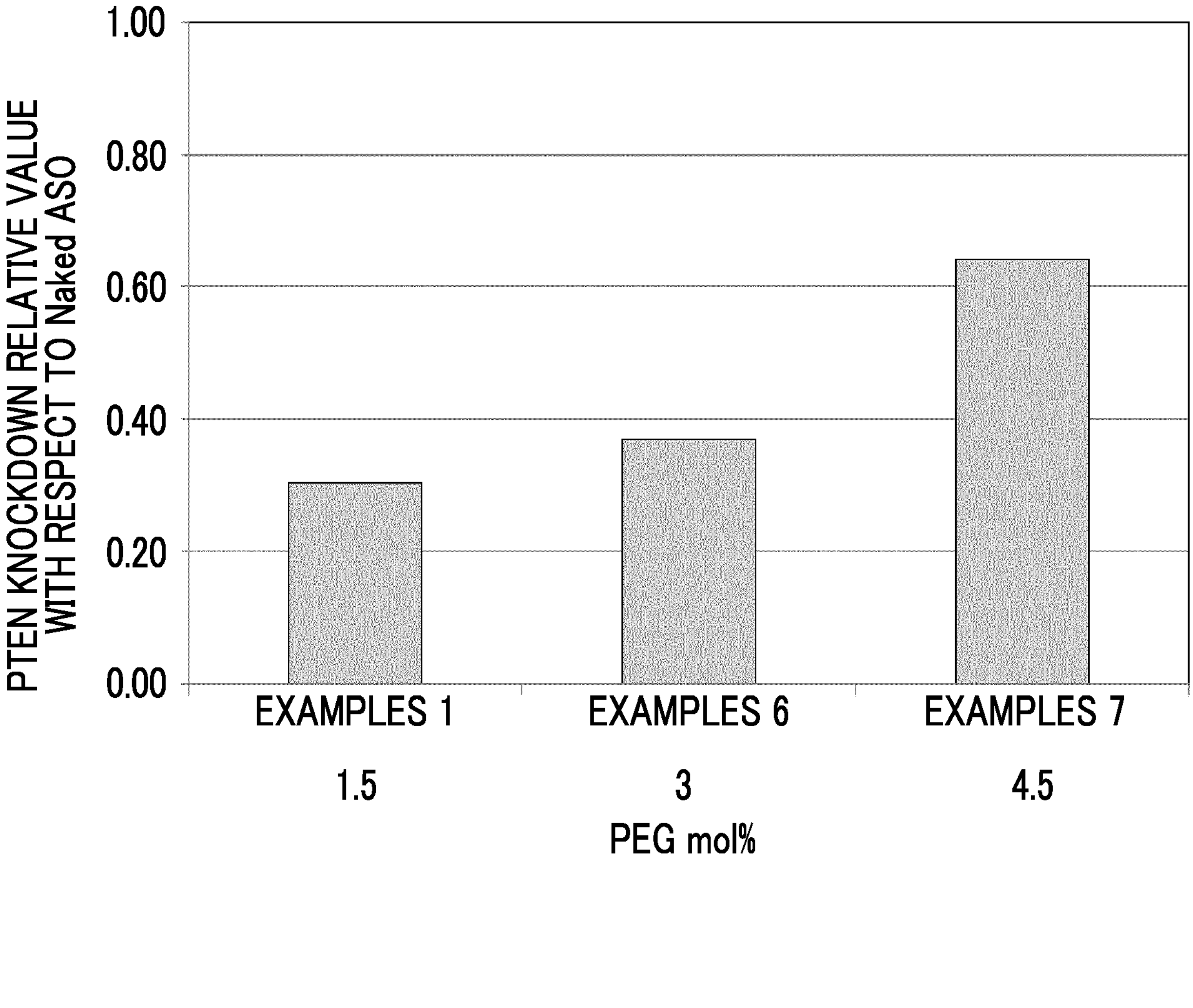

1.00
0.80
0.60
0.40
0.20
0.00
PTEN KNOCKDOWN RELATIVE VALUE
WITH RESPECT TO Naked ASO
EXAMPLES 1
EXAMPLES 6
EXAMPLES 7
1.5
3
4.5
PEG mol%

FIG. 4

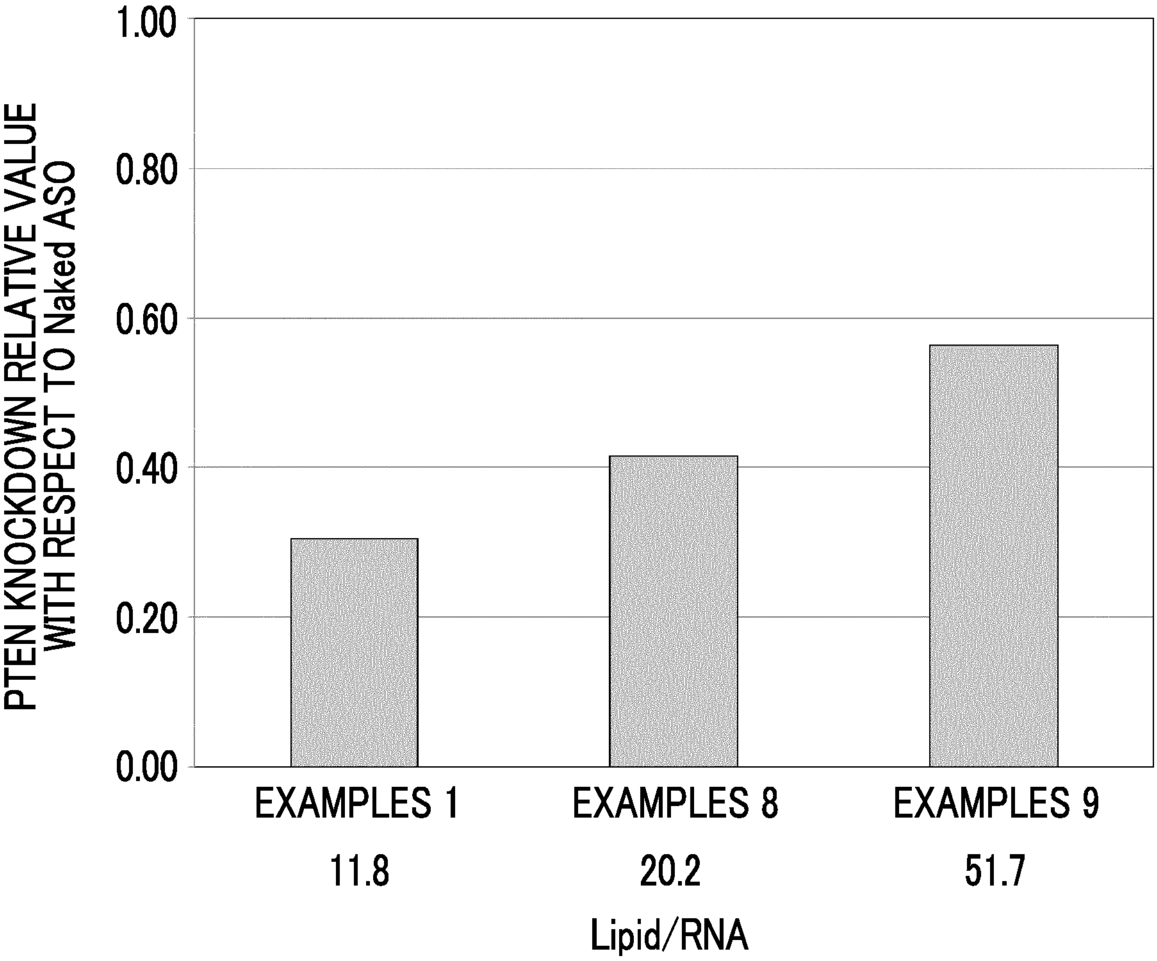
PTEN KNOCKDOWN RELATIVE VALUE
WITH RESPECT TO Naked ASO
1.00
0.80
0.60
0.40
0.20
0.00
EXAMPLES 1
11.8
EXAMPLES 8
20.2
EXAMPLES 9
51.7
Lipid/RNA

FIG. 5

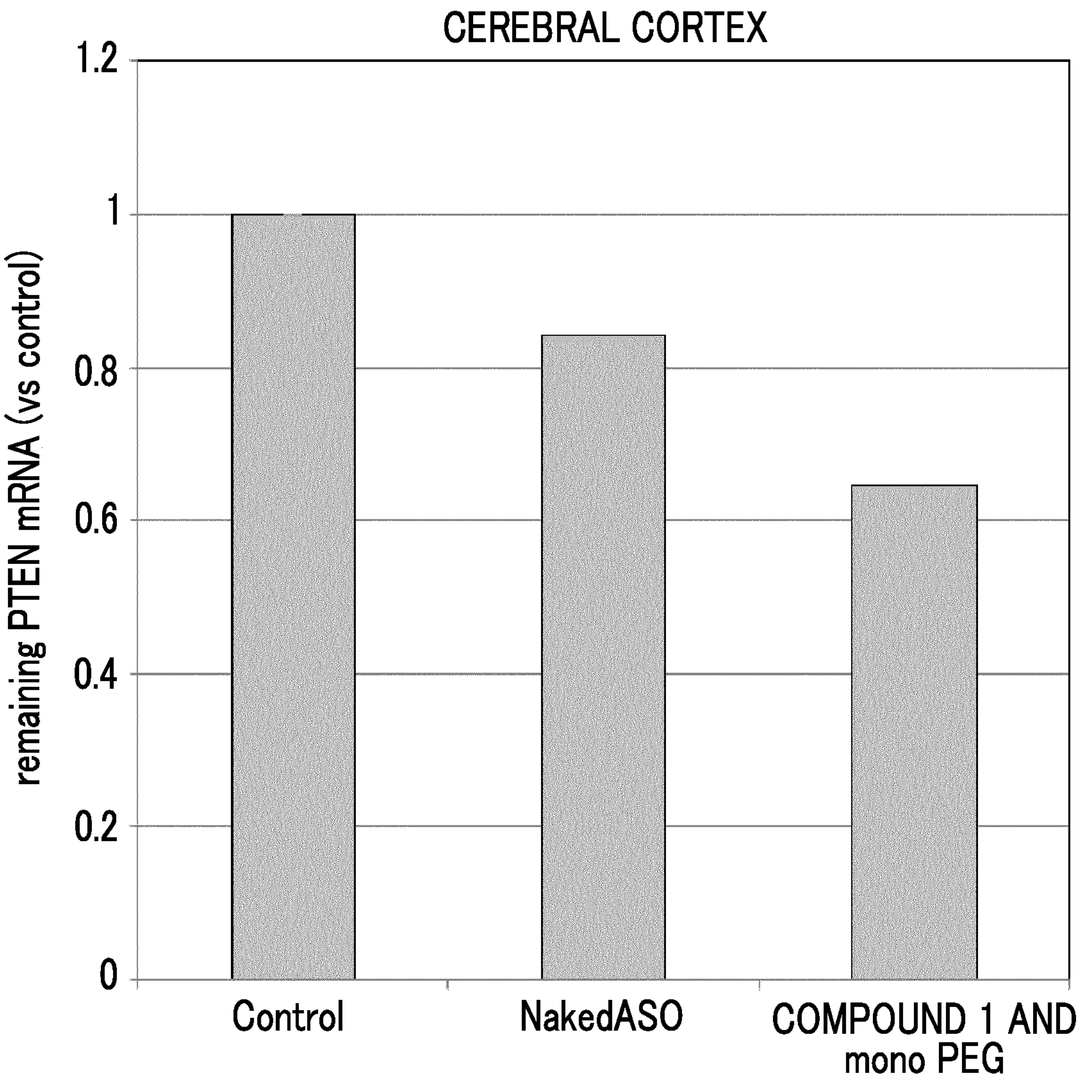
CEREBRAL CORTEX
remaining PTEN mRNA (vs control)
1.2
1
0.8
0.6
0.4
0.2
0
Control
NakedASO
COMPOUND 1 AND mono PEG

FIG. 6

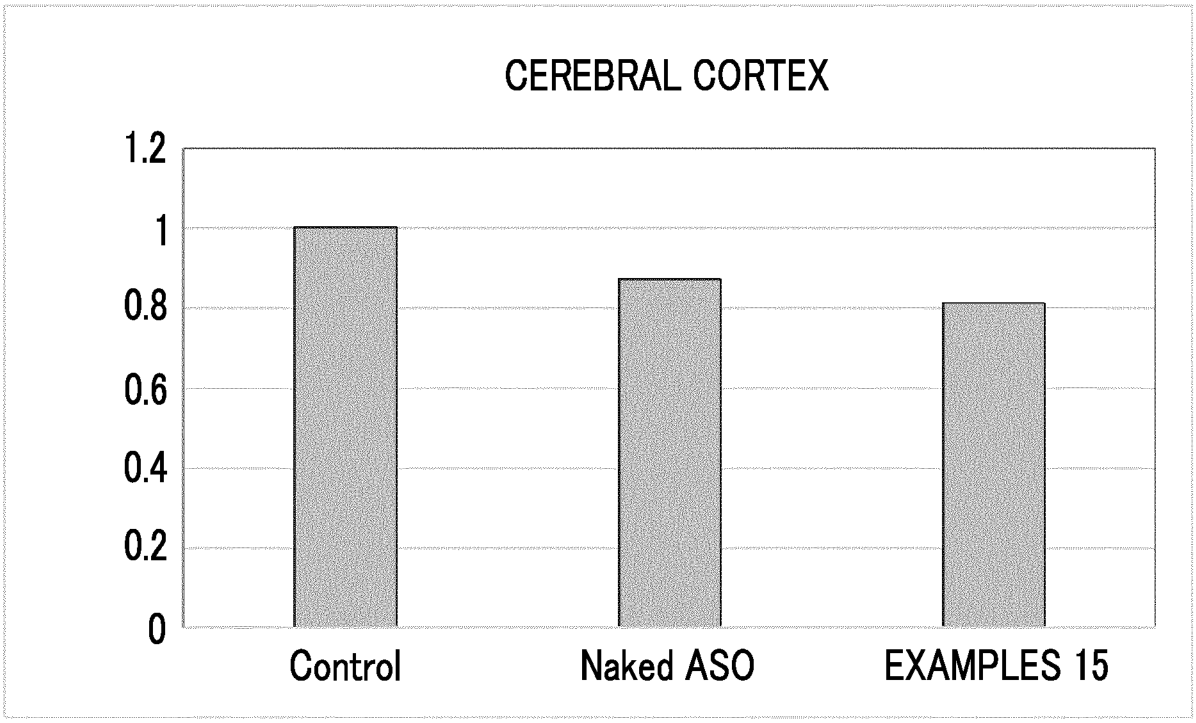
CEREBRAL CORTEX
1.2
1
0.8
0.6
0.4
0.2
0
Control
Naked ASO
EXAMPLES 15

## INTERNATIONAL SEARCH REPORT

International application No.
**PCT/JP2021/033635**

**A. CLASSIFICATION OF SUBJECT MATTER**

***A61K 31/7088***(2006.01)i; ***A61K 47/10***(2006.01)i; ***A61K 47/18***(2006.01)i; ***A61K 47/24***(2006.01)i; ***A61K 47/28***(2006.01)i; ***A61K 48/00***(2006.01)i; ***A61P 25/00***(2006.01)i; ***A61P 43/00***(2006.01)i; ***C12N 15/88***(2006.01)i
FI: A61K31/7088; A61P43/00 111; A61P25/00; A61K48/00; A61K47/18; A61K47/10; A61K47/28; A61K47/24; C12N15/88 Z ZNA

According to International Patent Classification (IPC) or to both national classification and IPC

**B. FIELDS SEARCHED**

Minimum documentation searched (classification system followed by classification symbols)

A61K31/7088; A61K47/10; A61K47/18; A61K47/24; A61K47/28; A61K48/00; A61P25/00; A61P43/00; C12N15/88

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
|---|---|---|
| Y | WO 2019/235635 A1 (FUJIFILM CORP.) 12 December 2019 (2019-12-12) claim 1, paragraphs [0008], [0088]-[0097], [0108]-[0112], [0348], [0349] | 1-19 |
| Y | WO 2006/080452 A1 (KYOWA HAKKO KOGYO CO., LTD.) 03 August 2006 (2006-08-03) example 12 | 1-19 |
| Y | JP 2006-290894 A (ASAHI KASEI CHEMICALS CORP.) 26 October 2006 (2006-10-26) paragraphs [0050]-[0054] | 1-19 |
| Y | JP 2006-321800 A (L'OREAL SA) 30 November 2006 (2006-11-30) paragraphs [0035]-[0039] | 1-19 |

☐ Further documents are listed in the continuation of Box C.  ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **18 October 2021** | **09 November 2021** |


| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |


Form PCT/ISA/210 (second sheet) (January 2015)

**INTERNATIONAL SEARCH REPORT**
**Information on patent family members**

International application No.
**PCT/JP2021/033635**

| Patent document cited in search report | Publication date (day/month/year) | Patent family member(s) | Publication date (day/month/year) |
|---|---|---|---|
| WO 2019/235635 A1 | 12 December 2019 | US 2021/0085604 A1<br>claim 1, paragraphs [0009], [0354]-[0375], [0390]-[0402], [1237], [1238]<br>EP 3805198 A1 | |
| WO 2006/080452 A1 | 03 August 2006 | US 2008/0145414 A1<br>example 12<br>EP 1842585 A1 | |
| JP 2006-290894 A | 26 October 2006 | (Family: none) | |
| JP 2006-321800 A | 30 November 2006 | US 2007/0003501 A1<br>paragraphs [0059]-[0078]<br>EP 1723972 A1<br>FR 2885813 A1<br>CA 2547127 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2010054401 A
- WO 2010144740 A
- WO 2010054405 A
- US 20130245107 A
- WO 2015095340 A
- WO 2009127060 A
- WO 2019235635 A
- JP 2019504002 A
- JP 5288254 B